Europäisches Patentamt

European Patent Office

Office européen des brevets

(19)

(11)  EP 1 299 563 B1

(12)  EUROPÄISCHE PATENTSCHRIFT

(45) Veröffentlichungstag und Bekanntmachung des Hinweises auf die Patenterteilung:
**14.12.2005  Patentblatt 2005/50**

(21) Anmeldenummer: 01954002.0

(22) Anmeldetag: **02.07.2001**

(51) Int Cl.[7]: **C12Q 1/68**

(86) Internationale Anmeldenummer:
**PCT/EP2001/007575**

(87) Internationale Veröffentlichungsnummer:
**WO 2002/002810 (10.01.2002 Gazette 2002/02)**

(54) **VERFAHREN ZUM QUALITATIVEN UND/ODER QUANTITATIVEN NACHWEIS VON MOLEKULAREN WECHSELWIRKUNGEN AUF SONDEN-ARRAYS**

METHOD FOR QUALITATIVE AND/OR QUANTITATIVE DETECTION OF MOLECULAR INTERACTIONS ON PROBE ARRAYS

PROCEDE DE DETECTION QUALITATIVE ET/OU QUANTITATIVE D'INTERACTIONS MOLECULAIRES SUR DES MICROPLAQUES

(84) Benannte Vertragsstaaten:
**AT BE CH CY DE DK ES FI FR GB GR IE IT LI LU MC NL PT SE TR**
Benannte Erstreckungsstaaten:
**LT LV SI**

(30) Priorität: **01.07.2000  DE 10033334**

(43) Veröffentlichungstag der Anmeldung:
**09.04.2003  Patentblatt 2003/15**

(73) Patentinhaber: **Clondiag Chip Technologies GmbH**
**07743 Jena (DE)**

(72) Erfinder:
 • **BICKEL, Ralf**
   **07743 Jena (DE)**
 • **EHRICHT, Ralf**
   **07749 Jena (DE)**
 • **ELLINGER, Thomas**
   **07743 Jena (DE)**
 • **ERMANTRAUT, Eugen**
   **07745 Jena (DE)**
 • **KAISER, Thomas**
   **07743 Jena (DE)**
 • **SCHULZ, Torsten**
   **07743 Jena (DE)**
 • **WAGNER, Gerd**
   **07743 Jena (DE)**

(74) Vertreter: **Maiwald Patentanwalts GmbH Elisenhof,**
**Elisenstrasse 3**
**80335 München (DE)**

(56) Entgegenhaltungen:
**EP-A- 0 267 521       WO-A-96/09532**
**WO-A-98/04740**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft eine Vorrichtung sowie ein Verfahren zum qualitativen und/oder quantitativen Nachweis von bestimmten molekularen Targets mit Hilfe von Sonden-Arrays.

Biomedizinische Tests basieren häufig auf der Feststellung der Wechselwirkung zwischen einem Molekül, das in bekannter Menge und Position vorhanden ist (der molekularen Sonde) und dem nachzuweisenden Molekül bzw. den nachzuweisenden Molekülen (dem molekularen Target). Moderne Tests werden in der Regel mit einer Probe parallel an einigen Sonden durchgeführt (D. J. Lockhart, E. A. Winzeler; Genomics, gene expression and DNA arrays; Nature 2000, 405, 827-836). Die Sonden werden hierbei üblicherweise in vorgegebener Art und Weise auf einer geeigneten, beispielsweise in WO 00/12575 beschriebenen Matrix immobilisiert (siehe z.B. US 5,412,087, WO 98/36827) bzw. synthetisch erzeugt (siehe z.B. US 5,143,854, US 5,658,734, WO 90/03382).

Der Nachweis einer solchen Wechselwirkung erfolgt üblicherweise folgendermaßen: Die Sonde bzw. die Sonden werden in vorgegebener Art und Weise an einer bestimmten Matrix fixiert. Die Targets werden in einer Lösung mit den Sonden in Kontakt gebracht und unter definierten Bedingungen inkubiert. In Folge der Inkubation findet zwischen Sonde und Target eine spezifische Wechselwirkung statt. Die entstehende Bindung ist deutlich stabiler als die Bindung von Molekülen, für die die Sonde unspezifisch ist. Anschließend wird das System mit entsprechenden Lösungen gewaschen, so daß die Moleküle, die nicht spezifisch gebunden sind, entfernt werden.

Zum Nachweis der Wechselwirkung zwischen Target und Sonde werden heute eine Vielzahl von Verfahren eingesetzt, von denen einige im folgenden beschrieben werden:

E. Lidell und I. Weeks, Antibody Technology, BIOS Scientific Publishers Limited, 1995, beschreiben die Markierung eines Targets mit einem Farbstoff bzw. einem Fluoreszenzfarbstoff und dessen Nachweis mit Hilfe eines Photometers bzw. eines Fluoreszenzdetektors. Die fluoreszenzoptische Detektion von Targets, die mit einem fluoreszierenden Marker versehen wurden, ist auch in F. Lottspeich, H. Zorbas, Bioanalytik, Spektrum Akademischer Verlag, Heidelberg, Berlin, 1998, beschrieben.

In Nature Biotechnology 1998, 16, 725-727 wird der Nachweis des Komplexes aus Target und Sonde durch Massenspektrometrie beschrieben. Ferner werden massensensitive Verfahren wie Oberflächenplasmonresonanz eingesetzt (J. M. Brockman et al., A multistep chemical modification procedure to create DNA Arrays on Gold surfaces for the study of Protein-DNA Interactions with surface plasmon resonance imaging, J. Am. Chem. Soc. 1999, 121, 8044-8051). Die US-Patentschrift 5,605,662 offenbart ein Verfahren zum direkten elektrischen Nachweis der Wechselwirkung. In DE 19543232 ist die Markierung des Targets mit Detektionskügelchen beschrieben, deren Anwesenheit nach der Wechselwirkung des Targets mit den Sonden optisch nachgewiesen wird.

Aus EP 0 063 810 ist ein Verfahren bekannt, bei dem auf einem festen porösen Substrat Targets in Form von Antigenen oder Immunglobulinen immobilisiert werden und durch Anwendung bekannter Techniken der Immunologie, insbesondere ELISA auf ihre Identität und Menge hin geprüft werden.

Zur Detektion molekularer Wechselwirkungen mit Hilfe von Sonden-Arrays auf festen Oberflächen sind verschiedene technische Ansätze beschrieben. Klassische Systeme beruhen auf dem Vergleich der Fluoreszenzintensitäten spektral selektiv angeregter, mit Fluorophoren markierter Targetmoleküle. Dazu sind verschiedene technische Lösungen bekannt, die sich hinsichtlich ihres optischen Aufbaus und der verwendeten Komponenten unterscheiden. Probleme und Limitationen solcher Aufbauten resultieren aus dem Signalrauschen (dem Hintergrund), das durch Effekte wie Bleichen und Quenching der verwendeten Farbstoffe, Autofluoreszenz der Medien, Assemblierungselementen und optischen Komponenten sowie Streuungen, Reflexionen und Fremdlicht im optischen Aufbau wesentlich bestimmt wird.

Daraus resultiert ein hoher technischer Aufwand zum Aufbau hochsensitiver Fluoreszenz-Detektoren zum qualitativen und quantitativen Vergleich von Sonden-Arrays. Insbesondere zum Screening mit mittleren und hohen Durchsätzen sind speziell angepaßte Detektionssysteme erforderlich, die einen gewissen Automatisierungsgrad besitzen.

Zur Optimierung von Standardpifluoreszenz-Aufbauten zum Auslesen molekularer Arrays sind CCD-basierte Detektoren bekannt, die zur Diskriminierung von optischen Effekten wie Streuung und Reflexionen die Anregung der Fluorophore im Dunkelfeld durch Auflicht oder Durchlicht realisieren (siehe z.B. C. E. Hooper et al., "Quantitative Photon Imaging in the Life Sciences Using Intensified CCD Cameras", Journal ofBioluminescence and Chemiluminescence (1990), S. 337-344.). Die Abbildung der Assays erfolgt dabei entweder in einer Belichtung oder durch ein Rastern unter Verwendung von höher auflösender Optik. Die Verwendung von multispektralen Belichtungsquellen ermöglicht einen relativ einfachen Zugang zu verschiedenen Fluorophoren durch die Verwendung verschiedener Anregungsfilter(-kombinationen). Nachteilig ist allerdings, daß Autofluoreszenz und systembedingte optische Effekte wie die Beleuchtungshomogenität über dem Assay komplizierte Beleuchtungsoptiken und Filtersysteme erfordern.

Beispielsweise in US 5,304,810 beschriebene kolifokale Scanning-Systeme beruhen auf der Selektion der Fluoreszenzsignale entlang der optischen Achse mittels zweier Pinholes. Daraus ergibt sich ein hoher Justageaufwand der Proben bzw. die Etablierung eines leistungsfähigen Autofokussystems. Solche Systeme sind in der technischen Lösung hochkomplex. Erforderliche Komponenten wie Laser, Pinholes, gegebenenfalls gekühlte Detektoren wie z.B. PMT, Avalanche-Dioden oder CCD, komplexe hochgenaue mechanische Translationselemente und Optiken müssen mit erheblichem Aufwand aufeinander optimiert und integriert werden (siehe z.B. US 5,459,325; US 5,192,980; US 5,834,758). Der Miniaturisierungsgrad und Preis ist durch die Vielzahl und Funktionalität der Komponenten limitiert.

Analysen basierend auf Sonden-Arrays werden somit zum derzeitigen Zeitpunkt in der Regel fluoreszenzoptisch ausgelesen (siehe A. Marshall und J. Hodgson, DNA Chips: An array ofpossibilities, Nature Biotechnology, 16,1998,27-31; G. Ramsay, DNA Chips: State of the Art, Nature Biotechnology, 16, Jan. 1998, 40-44). Nachteilig an den vorstehend beschriebenen Detektionsverfahren ist jedoch der hohe Signalhintergrund, der zu einer eingeschränkten Genauigkeit führt, der teilweise erhebliche technische Aufwand sowie die hohen Kosten, die mit den Nachweisverfahren verbunden sind.

Es besteht folglich ein Bedarf an hochintegrierten Arrays, mit denen mit relativ geringem technischen Aufwand die Wechselwirkung zwischen Sonden und Targets mit hoher Genauigkeit qualitativ und/oder quantitativ nachgewiesen werden kann.

Die Erhöhung der Selektivität und der Zugang zu alternativen Komponenten motiviert die Etablierung alternativer Imaging-Technologien wie Fluoreszenz-Polarisation und zeitaufgelöste Fluoreszenz für festkörpergebundene Assays. Solche Lösungen insbesondere für hochintegrierte Arrays liegen derzeit jedoch nur konzeptionell vor. Der Effekt der Verdrehung der Polarisationsachse durch polarisiert angeregte Fluorophore wird zur Quantifizierung im Mikrotiter-Format angewandt. Es gibt ferner Ansätze, durch die Verwendung entsprechend modifizierter Polymerfolien als Polarisationsfilter kostengünstige Systeme mit hohem Durchsatz (HTS-Systeme) aufzubauen (siehe I. Gryczcynski et al., Polarisation sensing with visual detection", Anal. Chem. 1999, 71, 1241-1251). Die zur Verfügung stehenden Lichtmengen und Detektoren machen derzeit allerdings eine Umsetzung auf Mikroarrays schwierig Ein derartiger Aufbau würde die Integration von Lichtquellen (z.B. Laser, LED, Hochdrucklampen), Polarisationsfiltern (u.U. beschichtete Polymerfolien) und Detektoren (CCD-, CMOS-Kamera) erfordern, eine entsprechende Lösung ist bislang nicht bekannt.

Neuere Entwicklungen nutzen die Fluoreszenz von anorganischen Materialien, wie zum Beispiel von Lanthaniden (M. Kwiatowski et al., Solid-phase synthesis of chelate-labelled oligonucleotides: application in triple-color ligase-mediated gene analysis, Nucleic Acids Research, 1994, 22, 13) und Quantendots (M. P. Bruchez et. al., Semiconductor Nanocrystals as Fluorescent Biological Labels, Science 1998, 281, 2013). Die Ausnutzung der spezifischen Fluoreszenzlebensdauer von Fluorophoren im ns-Bereich zu deren selektiven Quantifizierung ist sehr aufwändig und wird trotz der Spezifität in dieser ortsaufgelösten Applikation kommerziell nicht verwendet. Farbstoffe mit langer Emissionsdauer im µs-Bereich wie Lanthanid-Gelate erfordert eine Umwandlung der Farbstoffe in eine mobile Phase, so daß eine ortsaufgelöste Detektion nicht möglich ist.

Die Verwendung von Mikropartikeln, die aus ihrer Verwendung in Fernsehröhren bekannt sind (siehe F. van de Rijke et al., Up-Converting Phosphors: A New Reporter Technology for Nucleic Acid Microarrays, European EC Meeting on Cytogenetics (2000) Bari, Italien), als biologische Marker hat bezüglich Sensitivität und Miniaturisierbarkeit des Aufbaus der Detektionstechnik großes Potential, zumal anregungsseitig Lichtquellen aus der Datenübertragung Verwendung finden (980 nm Dioden-Laser). Diese Technologie ist jedoch z.Zt. nicht kommerziell zur Detektion von Target/Sonden-Wechselwirkungen auf Arrays verfügbar. Ein Detektor würde Komponenten zur Lichtemission (z.B. Laser, LED, Hochdrucklampen), ein System zur Modulierung des Anregungs- und Detektionslichtes (z.B. Chopperscheiben, elektronische Shutter) und Detektion des zeitverzögerten Signals (z.B. CCD-, CMOS-Kamera) beinhalten. Als grundsätzlich problematisch erweist sich jedoch in der Regel die geringe Kompatibilität der Partikel mit biologischen Proben.

Der Einsatz von Arrays mit immobilisierten Targets auf einem porösen Substrat hat im Gegensatz zum Einsatz von Sonden-Arrays den prinzipiellen Nachteil, daß bei jeder erfolgenden Analyse zunächst ein Array mit dem zu untersuchenden Material erzeugt werden muß, daß dann mit einem Satz bekannter Sonden in Verbindung gebracht wird. Der diagnostische Einsatz ist damit stark eingeschränkt, da Arrays häufig neu hergestellt werden müssen. Da das Material in der Regel biologischer Herkunft ist, sind Unterschiede zwischen Chargen unvermeidlich. Der Einsatz poröser Substrate limitiert die maximal erreichbare Auflösung der erzeugten Arrays, da die aufgebrachte Flüssigkeit sich lateral ausbreiten kann, so sind Größen einzelner Elemente auf porösen Materialien mit heute bekannter Depositionstechnik unterhalb von 200 µm kaum zu realisieren.

Aufgabe der vorliegenden Erfindung ist es somit, die vorstehend genannten Probleme des Stands der Technik, die sich insbesondere durch den komplexen Aufbau des Detektionssystems, den hohen Signalhintergrund u.a. durch Bleichen des Signals und die mangelnde Kompatibilität des Assays mit dem Testsystem ergeben, zu überwinden.

Insbesondere ist es eine Aufgabe der vorliegenden Erfindung, ein Verfahren bzw. eine Vorrichtung zur Verfügung zu stellen, mit dem molekulare Wechselwirkungen zwischen Sonden und Targets auf Sonden-Arrays mit hoher Genauigkeit und auf einfache und kostengünstige Weise qualitativ und/oder quantitativ nachgewiesen werden können.

Eine weitere Aufgabe der vorliegenden Erfindung ist es, daß eine hohe dynamische Auflösung bei der Detektion erreicht wird, d.h. der Nachweis schwacher Sonden/Target-Wechselwirkungen neben starken Signalen gewährleistet bleibt.

Diese und weitere Aufgaben der vorliegenden Erfindung werden durch die Bereitstellung der in den Patentansprüchen gekennzeichneten Ausführungsformen gelöst.

Überraschenderweise wurde jetzt gefunden, daß molekulare Wechselwirkungen zwischen Sondenmolekülen (im folgenden als Sonden bezeichnet) und Targetmolekülen (im folgenden als Targets bezeichnet) auf Sondenarrays durch ein einfaches und kostengünstiges Verfahren mit hoher Genauigkeit nachgewiesen werden können. Der Nachweis erfolgt bei dem erfindungsgemäßen Verfahren durch eine Reaktion, die ein Produkt mit einem bestimmtem Löslichkeitsprodukt liefert, das eine Präzipitation bzw. eine Niederschlagbildung des Produkts auf einem Array-Element des Sonden-Arrays, an dem eine Wechselwirkung zwischen Sonde und Target stattgefunden hat, zur Folge hat.

Vorzugsweise sind die gebundenen Targets mit einer Markierung versehen, die die Reaktion eines löslichen Substrats zu einem schwerlöslichen Niederschlag auf dem Array-Element katalysiert, an dem eine Sonden/Target-Wechselwirkung stattgefunden hat, bzw. die als Kristallisationskeim für die Umwandlung eines löslichen Substrats zu einem schwerlöslichen Niederschlag auf dem Array-Element wirkt, an dem eine Sonden/Target-Wechselwirkung stattgefunden hat.

Der Einsatz von Sonden-Arrays auf nicht porösen Trägern erlaubt auf diese Weise die simultane qualitative und quantitative Analyse einer Vielzahl von Sonden/Target-Wechselwirkungen, wobei einzelne Sonden-Elemente mit einer Größe von $\leq 1000\ \mu m$, vorzugsweise von $\leq 100\ \mu m$ und besonders bevorzugt $\leq 50\ \mu m$ realisiert werden können.

In der Immunzytochemie und bei immunologischen Mikrotiterplatten-basierten Tests ist der Einsatz von enzymatischen Markierungen bekannt (siehe E. Lidell und I. Weeks, Antibody Technology, BIOS Scientific Publishers Limited, 1995). So katalysieren beispielsweise Enzyme den Umsatz eines Substrats in ein schwerlösliches, in aller Regel gefärbtes Produkt. Eine weitere Möglichkeit des Nachweises molekularer Wechselwirkungen auf Arrays besteht im Einsatz von Metallmarkierungen. Hierbei werden kolloidales Gold oder definierte Goldcluster mit den Targets gekoppelt, ggf. über bestimmte Vermittlermoleküle wie Streptavidin, und durch nachfolgende Reaktion mit unedleren Metallen wie z.B. Silber verstärkt.

Die relative Quantifizierung der Konzentration der gebundenen Targets auf einem Sonden-Array durch Nachweis eines Präzipitats bzw. eines Niederschlags erfolgt bei dem erfindungsgemäßen Verfahren über die Konzentration der mit den Targets gekoppelten Markierungen, die die Reaktion eines löslichen Substrats zu einem schwerlöslichen Niederschlag auf dem Array-Element, an dem eine Sonden/Target-Wechselwirkung stattgefunden hat, katalysieren bzw. als Kristallisationskeim für derartige Reaktionen wirken. Beispielsweise beträgt im Fall von mit Nanogold markierten HPLC-gereinigten Oligonukleotidsonden das Verhältnis von gebundenem Target zu Goldpartikel 1:1. In anderen Ausführungsformen der vorliegenden Erfindung kann es Vielfaches oder auch einen Bruchteil davon betragen.

Die Konzentration der mit den Targets gekoppelten Markierungen (c(M)) steht mit der Konzentration des deponierten Niederschlags (c(N)) auf dem Array-Element in folgender Beziehung:

c(M) = [F*c(N)]/t; wobei F eine Kurvenfunktion ist, die den zeitlichen Verlauf der Präzipitationsreaktion charakterisiert und t die Zeit ist.

F ist aus dem zeitlichen Verlauf der Reaktion bestimmbar. Falls der zeitliche Verlauf der Niederschlagsbildung als lineare Funktion beschrieben werden kann (F = konstant), ist eine eindeutige Korrelation der gebildeten Niederschlagsmenge mit der Konzentration an gebundenen Targetmolekülen möglich, da dann c(N)/t ein Maß für c(M) und damit auch für die Konzentration der mit den Markierungen gekoppelten Targets ist. Folglich ist nur bei Kenntnis des zeitlichen Verlaufs der Präzipitationsreaktion eindeutig eine relative Bestimmung der an den jeweiligen Array-Elementen gebundenen Targetkonzentrationen möglich ist.

Herkömmlicherweise wird nach einer bestimmten Zeit nach der Wechselwirkung der Targets mit dem auf dem Sondenarray angeordneten Sonden sowie nach Beginn der Reaktion, die zu einem Niederschlag auf Array-Elementen führt, an denen eine Wechselwirkung stattgefunden hat, ein Bild aufgenommen und den gemessenen, von dem Grad der Niederschlagsbildung abhängigen Grauwerten Konzentrationen zugeordnet. Diese Vorgehensweise führt allerdings für das jeweilige Array-Element nur in einem sehr schmalen Konzentrationsbereich zu zufriedenstellenden Werten - und ist damit auch für der Bewertung der Spezifität von Wechselwirkungen problematisch - , da die Niederschlagsbildung in hohem Maße nichtlinear erfolgt. Insbesondere umfaßt der zeitliche Verlauf der Niederschlagsbildung einen exponentiellen Anstieg der Niederschlagsbildung mit der Zeit sowie ein darauffolgendes Sättigungsniveau. Lediglich Grauwerte aus dem Bereich des exponentiellen Anstiegs der Niederschlagsbildung mit der Zeit lassen eine Korrelation mit der Menge an gebundenen Targets zu, während das bei der Niederschlagsbildung auf einem Array-Element nach einer bestimmten, von der jeweiligen Sonden/Target-Wechselwirkung abhängigen und damit für jedes Array-Element unterschiedlichen Zeit erreichte Sättigungs-Niveau einer Quantifizierung nach Abschluß der Niederschlagsbildungs-Reaktion entgegensteht. Es ist nicht möglich, die Experimentparameter so zu gestalten, daß zweifelsfrei sichergestellt ist, daß das Sättigungsniveau auf keinem der Array-Elemente erreicht ist, da die Reaktionsgeschwindigkeit in starkem Maße von Temperatur, Licht, Salzkonzentration, pH und weiteren Faktoren abhängt.

Bei Aufnahme lediglich eines Bildes kann folglich nicht gewährleistet werden, daß sich die Niederschlagsbildungs-Reaktionen auf allen Array-Elementen in dem exponentiellen Bereich der Abhängigkeit der Niederschlagsbildung von der Zeit befinden. Somit werden Signalintensitäten, beispielsweise Grauwerte, die von Array-Elementen erhalten werden, bei denen sich die Niederschlagsreaktion bereits im Sättigungsbereich befindet, im Vergleich zu Signalintensitäten von Array-Elementen, die sich noch im exponentiellen Bereich der Niederschlagsbildung befinden, verfälscht dargestellt.

Um die vorstehend beschriebenen Nachteile zu überwinden wird durch die vorliegende Erfindung ein Verfahren zum qualitativen und/oder quantitativen Nachweis von Targets in einer Probe durch molekulare Wechselwirkungen zwischen Sonden und Targets auf Sonden-Arrays bereitgestellt, das die folgenden Schritte umfaßt:

a) Herstellung eines Sondenarrays mit an definierten Stellen immobilisierten Sonden;
b) Wechselwirkung der Targets mit den auf dem Sondenarray angeordneten Sonden;
c) Durchführung einer Reaktion, die zu einem Niederschlag an Array-Elementen führt, an denen eine Wechselwirkung erfolgt ist;
d) Detektion des zeitlichen Verlaufs der Niederschlagsbildung an den Array-Elementen in Form von Signalintensitäten;
e) Bestimmung einer virtuellen Signalintensität für ein Array-Element anhand einer Kurvenfunktion, die die Niederschlagsbildung als Funktion der Zeit beschreibt.

Zur Beschreibung der vorliegenden Erfindung werden die folgenden Definitionen verwendet:

Unter einer molekulare Sonde wird im Rahmen der vorliegenden Erfindung ein Molekül verstanden, daß zum Nachweis anderer Moleküle durch ein bestimmtes, charakteristisches Bindungsverhalten bzw. eine bestimmte Reaktivität verwandt wird.

Unter einem Sonden-Array wird im Rahmen der vorliegenden Erfindung eine Anordnung von molekularen Sonden auf einer Oberfläche verstanden, wobei die Position einer jeden Sonde separat bestimmt ist.

Unter einem Array-Element wird im Rahmen der vorliegenden Erfindung ein für die Deposition einer molekularen Sonde bestimmtes Areal auf einer Oberfläche verstanden, die Summe aller belegten Array-Elemente ist das Sonden-Array.

Unter einer Mikrotiterplatte wird im Rahmen der vorliegenden Erfindung eine Anordnung von Reaktionsgefäßen in einem bestimmten Raster verstanden, die die automatisierte Durchführung einer Vielzahl von biologischen, chemischen und labormedizinischen Tests gestattet.

Unter einem Target wird im Rahmen der vorliegenden Erfindung das mit einer molekularen Sonde nachzuweisende Molekül verstanden.

Unter HTS (engl.: high throughput screening) wird im Rahmen der vorliegenden Erfindung eine systematische Wirkstoffsuche mit hohem Durchsatz verstanden.

Unter einem Substrat wird im Rahmen der vorliegenden Erfindung ein im Reaktionsmedium gelöst vorliegendes Molekül bzw. eine Kombination von Molekülen verstanden, die mit Hilfe eines Katalysators bzw. eines Kristallisationskeims und eines reduzierenden Agens lokal abgeschieden wird.

Unter einem Träger wird im Rahmen der vorliegenden Erfindung ein Festkörper verstanden, auf dem das Sonden-Array aufgebaut ist.

Unter einer Markierung wird im Rahmen der vorliegenden Erfindung eine mit dem Target gekoppelte Gruppe verstanden, die die Reaktion eines löslichen Substrats zu einem schwerlöslichen Niederschlags katalysiert bzw. als Kristallisationskeim für die Umwandlung eines löslichen Substrats in einen schwerlöslichen Niederschlag wirkt.

Unter einer virtuellen Signalintensität wird im Rahmen der vorliegenden Erfindung ein Wert verstanden, der die Wechselwirkung zwischen Sonde und Target auf einem Array-Element und damit die Menge an gebundenen Targets quantifiziert und anhand einer Kurvenfunktion bestimmt wird, die die Niederschlagsbildung als Funktion der Zeit beschreibt.

Wesentliches Merkmal des erfindungsgemäßen Verfahrens ist die Bestimmung einer virtuellen Signalintensität für ein Array-Element in Abhängigkeit des zeitlichen Verlaufs der Niederschlagsbildung. Die Niederschlagsbildung als Funktion der Zeit auf einem Array-Element wird erfindungsgemäß vorzugsweise durch eine Kurvenfunktion beschrieben, anhand derer eine virtuelle Signalintensität bestimmt wird. Diese virtuelle Signalintensität ist aufgrund der Berücksichtigung des zeitlichen Verlaufs der Niederschlagsbildung ein unverfälschtes Maß für die Menge an gebundenen Targets.

Insbesondere können erfindungsgemäß Teilbereiche oder auch der gesamte Bereich des zeitlichen Verlaufs der Niederschlagsbildung als Regressionsgerade beschreiben werden. Die virtuelle Signalintensität wird bei dieser Ausführungsform in Abhängigkeit von der Steigung der Regressionsgerade bestimmt. Diese Steigung ist ein direktes Maß für die Konzentration der gebundenen Targets, d.h. je größer die Steigung der Regressionsgerade ist, desto mehr Targets sind gebunden. Bei Betrachtung aller Array-Elemente unter den gleichen Bedingungen ist der über die Zeit resultierende Anstieg der Niederschlagsbildung auf jedem einzelnen Array-Element charakteristisch für die Konzentration und für den jeweiligen Versuch normiert auf die vorherrschenden Bedingungen. Somit ist eine genaue Bestimmung der relativen quantitativen Menge an gebundenen Targets gewährleistet.

Bei einer besonders bevorzugten Ausführungsform der vorliegenden Erfindung wird die Regressionsgerade im Bereich des exponentiellen Anstiegs der Niederschlagsbildung mit der Zeit auf einem Array-Element ermittelt.

Bei einer Ausführungsform der vorliegenden Erfindung entspricht die Regressionsgerade einer Tangente an die Kurvenfunktion, mit der die Niederschlagsbildung als Funktion der Zeit beschreiben werden kann, durch den Wendpunkt der Kurvenfunktion. Der Wendepunkt der Kurvenfunktion werden durch Bestimmung des Maximums der 1. Ableitung der Kurvenfunktion ermittelt.

Bei einer alternativen Ausführungsform der vorliegenden Erfindung wird die Regressionsgerade ermittelt, in dem die Scheitelpunkte der Kurvenfunktion, mit der die Niederschlagsbildung als Funktion der Zeit beschreiben werden kann, durch eine Gerade verbunden werden. Die Scheitelpunkte der Kurvenfunktion werden durch Bestimmung der Maxima der 2. Ableitung der Kurvenfunktion ermittelt.

Ferner kann durch Bestimmung einer virtuellen Signalintensität für jedes Array-Element in Abhängigkeit von dem zeitlichen Verlauf der Niederschlagsbildung und anschließende Umwandlung dieser virtuellen Signalintensitäten in ein analoges Bild der dynamische Meßbereich, d.h. der Umfang des Nachweisbereichs vervielfacht werden. Eine Erweiterung der Dynamik der Messung wird dadurch ermöglicht, daß nicht mehr die Farbtiefe des Detektorsystems als bestimmendes Element auftritt, sondern der über die Zeit gemessene Verlauf der Anlagerung des Präzipitats an die Oberfläche des jeweiligen Sondenarray-Elements. Über die Auswertung des Anstiegs der Präzipitationsreaktion ist es möglich, eine virtuelle Signalintensitäts- bzw. Grauwertverteilung zu bestimmen, und hiermit schließlich eine erweiterte Dynamik zu erreichen.

Das erfindungsgemäße Verfahren bietet den weiteren Vorteil, daß Detektionssysteme eingesetzt werden können, die nicht aufwändig und zusätzlich preiswert sind. Beispielsweise kann eine Kamera mit lediglich 8 bit, d.h. 256 Grauwerten, Grauwerttiefe verwendet werden, die nach Auswertung und Erstellung einer virtuellen Abbildung eine reale Tiefenschärfe von 24 bit (16777216 Grauwerte) oder 48 bit (33554432 Grauwerte) liefert und damit eine deutlich verbesserte Möglichkeit der simultanen Detektion von geringen und starken Wechselwirkungen von Targets mit Sonden auf einem Sonden-Array bietet.

Bei einer bevorzugten Ausführungsform der vorliegenden Erfindung liegt in der zu untersuchenden Probe ein Referenz-Target mit bekannter Konzentration vor, das mit mindestens einer Sonde des Sondenarrays wechselwirkt. Die dieser Sonden/Referenz-Target-Wechselwirkung entsprechende virtuelle Signalintensität, bestimmt in Abhängigkeit von dem Anstiegt der Niederschlagsbildung in Abhängigkeit von der Zeit, dient als Referenz für die Quantifizierung der übrigen Target-Konzentrationen entsprechend der jeweiligen virtuellen Signalintensitäten, die durch die Sonden/Target-Wechselwirkungen herbeigeführt werden, relativ zu der Konzentration der Referenz-Target-Konzentration.

Die zu untersuchenden Targets können in jeder Art von Probe, vorzugsweise in einer biologischen Probe vorliegen. Vorzugsweise werden die Targets vor ihrer Detektion und Quantifizierung durch das erfindungsgemäße Verfahren isoliert, gereinigt, kopiert und/oder amplifiziert.

Das im Rahmen der vorliegenden Erfindung eingesetzte Sondenarray mit an definierten Stellen immobilisierten Sonden wird nach herkömmlich bekannten Verfahren hergestellt. Ein Sondenarray gemäß der vorliegenden Erfindung umfaßt einen Träger, der die Bildung von Arrays mit Sonden auf seiner Oberfläche erlaubt. Ein derartiger Träger kann hergestellt werden aus Materialien, ausgewählt aus der Gruppe, bestehend aus Glas, Filtern, elektronischen Vorrichtungen, Polymeren, metallischen Materialien und dergleichen sowie Kombinationen dieser Materialien.

Vorzugsweise umfaßt der Array definierte Stellen, sog. Array-Elemente, die besonders bevorzugt in einem bestimmten Muster angeordnet sind, wobei jedes Array-Element üblicherweise nur eine Spezies an Sonden beinhaltet.

Bei einer weiteren bevorzugten Ausführungsform der vorliegenden Erfindung werden zur Detektion des zeitlichen Verlaufs der Niederschlagsbildung an den Array-Elementen die Signalintensitäten mindestens jede Minute, vorzugsweise alle 30 Sekunden, besonders bevorzugt alle 10 Sekunden aufgenommen. Daneben sind aber auch andere Zeitabstände für die Aufnahme der Signalintensitäten denkbar, mit der Maßgabe, daß die zeitliche Abhängigkeit der Niederschlagsbildung eindeutig bestimmt werden kann und damit beispielsweise die Steigung der Regressionsgerade im exponentiellen Bereich als Maß für die jeweilige Konzentration an gebundenen Targets abgeleitet werden kann.

Die virtuelle Signalintensität für ein Array-Elemente wird beispielsweise durch Multiplikation der detektierten Signalintensität zu einem bestimmten Zeitpunkt, vorzugsweise der Signalintensität der letzten Messung, mit der Steigung der für das Array-Element ermittelten Regressionsgerade sowie mit der Meßdauer zu dem bestimmten Zeitpunkt bestimmt. Selbstverständlich ist es bei dieser Ausführungsform für eine relative Quantifizierung erforderlich, daß der Zeitpunkt für die Detektion der Signalintensität für alle Array-Elemente identisch ist.

Voraussetzung für eine relative Quantifizierung der Konzentration der gebundenen Targets auf einem Sonden-Array durch Nachweis eines Präzipitats nach dem erfindungsgemäßen Verfahren ist ferner, daß die Targets mit Markierungen versehen sind, die die Reaktion eines löslichen Substrats zu einem schwerlöslichen Niederschlag auf dem Array-Element, an dem eine Sonden/Target-Wechselwirkung stattgefunden hat, katalysieren, bzw. als Kristallisationskeim für derartige Reaktionen wirken.

Dabei können bei einer Ausführungsform der vorliegenden Erfindung die Targets direkt mit derartigen Markierungen versehen sein.

Alternativ wird auf eine direkte Markierung der Targets verzichtet und die Markierung erfolgt über Sandwich-Hybridisierung oder Sandwich-Reaktionen mit der mit dem Target wechselwirkenden Sonde und einer markierten Verbindung. Beispiele für eine derartige Vorgehensweise sind:

- Sandwich-Hybridisierung mit einem zur Targetsequenz komplementären markierten Oligonukleotid.

- Sandwich-Hybridisierung von in Kettenform mit der Targetsequenz hybridisierenden markierten Oligonukleotiden: Unter in Kettenform mit der Targetsequenz hybridisierenden markierten Oligonukleotiden wird im Rahmen der vorliegenden Erfindung ein Satz von markierten Oligonukleotiden verstanden, von denen mindestens eines Komplementarität sowohl zur Targetsequenz als auch zu einem weiteren Oligonukleotid aufweist. Die anderen Oligonukleotide sind selbstkomplementär bzw. wechselseitig zueinander komplementär, so daß während der Hybridisierung eine Kette von an der Targetsequenz gebundenen markierten Oligonukleotiden entsteht.

- Sandwich-Hybridisierung mit einem zur Targetsequenz komplementären Oligonukleotid, das mit einer vielfach markierten Struktur, beispielsweise einem Dendrimer, beschrieben z.B. in WO 99/10362, gekoppelt ist.

Eine weitere bevorzugte Möglichkeit der Kopplung der Targets mit einer Markierung stellt das synthetische oder enzymatische Anfügen eines homopolymeren Bereichs, beispielsweise einer polyA-Seqeunz, an die Targets unter Bildung einer fortlaufenden Sequenz dar, wie es beispielsweise in US 6,103,474 beschrieben ist. Bei dieser Ausführungsform erfolgt die Markierung vorzugsweise über Sandwich-Hybridisierung mit einem zur Homopolymer-Sequenz komplementären markierten Oligonukleotid mit den vorstehend beschriebenen Variationen.

Bei einer weiteren bevorzugten Ausfiihrungsform der vorliegenden Erfindung erfolgt eine Signalamplifikation durch Amplifikation von Teilen der an die Targets angefügten Homopolymersequenz bei gleichzeitigem Einbau von markierten Basen, insbesondere bevorzugt über einen RCA-Mechanismus durch Verwendung eines zirkulären einzelsträngigen Templates, das Komplementarität zur Homopolymersequenz aufweist.

Die nachfolgende Tabelle 1 gibt, ohne den Anspruch zu erheben, vollständig zu sein, einen Überblick über eine Reihe von in Frage kommenden Reaktionen, die geeignet sind, um zu einem Niederschlag an Array-Elementen zu führen, an denen eine Wechselwirkung zwischen Target und Sonde erfolgt ist:

Tabelle 1

| Katalysator bzw. Kristallisationskeim | Substrat |
|---|---|
| Meerrettichperoxidase | DAB (3,3' -Diaminobenzidm) <br> 4-CN (4-Chlor-1-Napthol) <br> AEC (3-Amino-9-Ethylcarbazol) <br> HYR (p-Phenylendiamin-HCl und Pyrocatechol) <br> TMB (3,3',5,5'-Tetramethylbenzidin) <br> Naphtol/Pyronin |
| Alkalische Phosphatase | Bromchlorindoylphosphat (BCIP) und Nitrotetrazoliumblau (NBT) |
| Glucoseoxidase | t-NBT und m-PMS <br> (Nitrotetrazoliumblauchlorid und Phenazinmethosulfat |
| Goldpartikel | Silbernitrat <br> Silbertartrat |

Die Markierung von biologischen Proben mit Enzymen bzw. Gold, insbesondere nanokristallinem Gold ist hinlänglich beschrieben (siehe u.a. F. Lottspeich und H. Zorbas, Bioanalytik, Spektrum Akademischer Verlag, Heidelberg, Berlin, 1998; E. Lidell und I. Weeks, Antibody Technology, BIOS Scientific Publishers Limited, 1995).

Weitere Möglichkeiten für den Nachweis der Sonde/Target-Wechselwirkungen über unlösliche Präzipitate bei dem erfindungsgemäßen Verfahren sind beschrieben in: Immunogold-Silver Staining, Principles, Methods and Applications, Hrsg.: M.A.Hayat, 1995, CRC Press; Eur J Immunogenet 1991 Feb-Apr;18(1-2):33-55 HLA-DR, DQ and DP typing using PCR amplification and immobilized probes. Erlich H, Bugawan T, Begovich AB, Scharf S, Griffith R, Saiki R, Higuchi R, Walsh PS. Department of Human Genetics, Cetus Corp., Emeryville, California 94608; Mol Cell Probes 1993 Jun;7(3):199-207 A combined modified reverse dot-blot and nested PCR assay for the specific non-radioactive detection of Listeria monocytogenes. Bsat N, Batt CA. Department of Food Science, Cornell University, Ithaca, NY 14853. Immunogenetics 1990;32(4):231-41 Erratum in: Immunogenetics 1991;34(6):413 Rapid HLA-DPB typing using enzymatically amplified DNA and nonradioactive sequence-specific oligonucleotide probes. Bugawan TL, Begovich AB, Erlich HA. Department of Human Genetics, Cetus Corporation, Emeryville, CA 94608. Hum Immunol 1992 Dec; 35(4):215-22 Generic HLA-DRB1 gene oligotyping by a nonradioactive reverse dot-blot methodology. Eliaou JF, Palmade F, Avinens O, Edouard E, Ballaguer P, Nicolas JC, Clot J. Laboratory of Immunology, Saint Eloi Hospital, CHU Montpellier, France. J Immunol Methods 1984 Nov 30;74(2):353-60 Sensitive visualization of antigen-antibody reactions in dot and blot immune overlay assays with immunogold and immunogold/silver staining. Moeremans M, Daneels G, Van Dijck A, Langanger G, De Mey J. Histochemistry 1987;86(6):609-15 Non-radioactive in situ hybridization. A comparison of several immunocytochemical detection systems using reflection-contrast and electron microscopy. Cremers AF, Jansen in de Wal N, Wiegant J, Dirks RW, Weisbeek P, van der Ploeg M, Landegent JE.

Im Rahmen der vorliegenden Erfindung sind u.a. folgende Varianten für den Nachweis der Sonde/Target-Wechselwirkungen über unlösliche Präzipitate denkbar.

Bei einer Ausführungsform der vorliegenden Erfindung werden die Targets mit einem Katalysator, vor einem Enzym versehen, das die Umwandlung eines löslichen Substrats in ein unlösliches Produkt katalysiert. Die Reaktion, die zur Bildung eines Niederschlags an den Array-Elementen führt, ist in diesem Fall die Umwandlung eines löslichen Substrats in ein unlösliches Produkt in Gegenwart eines mit den Targets gekoppelten Katalysators, vorzugsweise Enzyms. Das Enzym wird vorzugsweise ausgewählt aus der Gruppe, bestehend aus Meerettichperoxidase, alkalischer Phosphatase und Glucoseoxidase. Das lösliche Substrat wird vorzugsweise ausgewählt aus der Gruppe, bestehend aus 3,3'-Diaminobenzidin, 4-Chlor-1-naphthol, 3-Amino-9-ethylcarbazol, p-Phenylendiamin-HCl/Pyrocatechol, 3,3', 5,5'-Tetramethylbenzidin, Naphthol/Pyronin, Bromchlorindoylphosphat, Nitrotetraazoliumblau und Phenazinmethosulfat. Beispielsweise wird ein farbloser löslicher Wasserstoffdonor, z.B. 3,3'-Diaminobenzidin, in Anwesenheit von Wasserstoffperoxid in ein unlösliches farbiges Produkt umgewandelt. Das Enzym Meerrettichperoxidase überträgt Wasserstoffionen aus den Donoren auf Wasserstoffperoxid unter Bildung von Wasser.

Bei einer bevorzugten Ausführungsform der vorliegenden Erfindung ist die Reaktion, die zur Bildung eines Niederschlags an den Array-Elementen führt, die Bildung eines metallischen Niederschlags ist. Besonders bevorzugt ist die zur Bildung eines Niederschlags an den Array-Elementen führende Reaktion die chemische Reduktion einer Silberverbindung, vorzugsweise Silbernitrat, Silberlactat, Silberacetat oder Silbertartrat, zu elementarem Silber. Als Reduktionsmittel wird vorzugsweise Formaldehyd und/oder Hydrochinon eingesetzt.

Besonders bevorzugt erfolgt die Ausfällung der metallischen Verbindung in Gegenwart von mit den Targets gekoppelten Metallclustern bzw. kolloidalen Metallpartikeln, insbesondere Goldclustern oder kolloidalen Goldpartikeln. D.h. in diesem Fall stellen die Metallcluster bzw. kolloidalen Metallpartikel die mit den Targets gekoppelten Markierungen dar. Beispielsweise wird Silbernitrat in elementares Silber umgesetzt, wobei sich Silberionen aus der Lösung an Gold als Kristallisationskeim anlagern und in einem zweiten Schritt mit Hilfe eines Reduktionsmittels wie z.B. Formaldehyd) reduziert werden. Hierbei entsteht ein unlöslicher Niederschlag elementaren Silbers.

Bei einer alternativen Ausführungsform erfolgt die Ausfällung der metallischen Verbindung in Gegenwart von mit den Targets gekoppelten Polyanionen. Wenn es sich beim Target selbst nicht um ein Poly-Anion handelt, dann besteht die Möglichkeit ein solches zur Keimbildung einzusetzen. Das mit einem Poly-Anion markierte Target wird beispielweise einer Silbernitratlösung ausgesetzt. Dabei lagern sich die Silber-Kationen am Poly-Anion selektiv an. Danach werden mit einem Reduktionsmittel Silberionen in elementares Silber umgewandelt.

Die Kopplung der Enzyme bzw. Katalysatoren bzw. Metallcluster bzw. kolloidalen Metallpartikel bzw. Polyanionen an die Targets kann direkt oder über an die Targets gekoppelte Ankermoleküle erfolgen. Grundsätzlich besteht keine Notwendigkeit, das Target direkt mit den oben beschriebenen Markierungen zu versehen. Es besteht auch die Möglichkeit, über geeignete Ankermoleküle, z.B. Streptavidin, die an das Target gekoppelt werden, eine nachfolgende Ankopplung der Markierung zu erreichen.

Ein Konjugat bestehend aus dem jeweiligen Katalysator bzw. Kristallisationskeim und einem spezifischen Bindungspartner für das Ankermolekül erlaubt ebenfalls die Durchführung der oben beschriebenen Prozeduren. Die Reaktion, die zur Bildung eines Niederschlags an den Array-Elementen führt, ist dann die Bindung eines spezifischen Bindungspartners an ein an das Target gekoppeltes Ankermolekül darstellt.

Derartige Bindungspartner/Ankermolekül-Paare werden vorzugsweise ausgewählt aus der Gruppe, bestehend aus Biotin/Avidin bzw. Streptavidin bzw. Anti-Biotin-Antikörper, Digoxigenin/Antidigoxigenin-Immunoglobulin, FITC/Anti-FITC-Immunoglobulin und DNP/Anti-DNP-Immunoglobulin.

In jeder der vorstehend beschriebenen Ausführungsformen wird katalytisch ein lösliches Substrat in ein unlösliches und ausfallendes Produkt umgesetzt. Aufgrund der Nähe zur Oberfläche wird das Produkt unmittelbar an der Oberfläche abgeschieden und bildet einen festen, für diverse Wäschen unempfindlichen Niederschlag.

Ferner ist es im Rahmen der vorliegenden Erfindung möglich, daß die Markierungen, insbesondere die Enzyme bzw. Metallcluster bzw. kolloidalen Metallpartikel bzw. Polyanionen, an die Targets vor, während oder nach der Wechselwirkung mit den Sonden gekoppelt werden.

Bei einer bevorzugtn Ausführungsform der vorliegenden Erfindung ist die Wechselwirkung zwischen dem Target und der Sonde eine Hybridisierung zwischen zwei Nukleotidsequenzen. Die Hybridisierung der Targets mit den auf einem Sonden-Array angeordneten Sonden findet nach einem der bekannten Standardprotokolle statt (siehe u.a. Lottspeich und Zorbas, 1998).

Die entstandenen Hybride können durch kovalente Bindung, beispielsweise über Psoralen-Interkalation und nachfolgendes "Cross-Linking", oder wie in US 4,599,303 beschrieben, durch nichtkovalente Bindung, beispielsweise durch Bindung von Interkalatoren, stabilisiert werden.

Im Anschluß an die Hybridisierung der Targets mit den auf einem Sonden-Array angeordneten Sonden bzw. der Markierung der hybridisierten Targets erfolgt üblicherweise ein Waschschritt, mit dem unspezifisch und dadurch schwächer gebundene Komponenten entfernt werden.

Alternativ ist die Wechselwirkung zwischen dem Target und der Sonde eine Reaktion zwischen einer Antigenstruktur und dem entsprechenden Antikörper oder einem hypervariablen Abschnitt davon oder eine Reaktion zwischen einem Rezeptor und einem entsprechenden Liganden ist.

Die Bindung oder Erkennung der Targets durch spezifische Sonden ist üblicherweise ein spontane nicht-kovalente Reaktion unter optimalen Bedingungen. Davon umfaßt sind ebenfalls nicht-kovalente chemische Bindungen. Die Zusammensetzung des Mediums sowei weitere chemische und physikalische Faktoren beeinflussen die Geschwindigkeit und Stärke der Bindung. So erniedrigen beispielsweise bei der Nukleinsäure-Erkennung eine niedrigere Stringenz und höhere Temperaturen die Rate und Stärke der Bindung zwischen zwei nicht perfekt komplementären Strängen. Die Optimierung der Bindungsbedingungen ist für Antigen/Antikörper- oder Ligand/Rezeptor-Wechselwirkungen ebenfalls erforderlich, die Bindungsbedingungen sind aber üblicherweise weniger spezifisch.

Der Nachweis der Gegenwart eines Niederschlags auf einem Array-Element erfolgt bei einer Ausfiihrungsform der vorliegenden Erfindung durch Reflexion, Absorption oder Diffusion eines Lichtstrahls, vorzugsweise eines Laserstrahls oder einer Leuchtdiode, durch den Niederschlag. Aufgrund seiner granularen Form modifiziert der Niederschlag die Reflexion eines Lichtstrahls. Ferner führt der Niederschlag zu einer starken Lichtdiffusion die durch herkömmliche Detektionsvorrichtungen aufgezeichnet werden kann. Falls der Niederschlag wie beispielsweise der Silberniederschlag als dunkle Oberfläche erscheint, kann auch die Absorption von Licht detektiert und aufgezeichnet werden. Die Auflösung der Detektion hängt dann von der Anzahl der Pixel der Kamera ab.

Beispielsweise kann der Nachweis der durch die spezifische Reaktion verstärkten Bereiche mittels eines sehr einfachen optischen Aufbaus im Durchlicht (Kontrast durch Abschattung) bzw. Auflicht (Kontrast durch Reflexion) erfolgen. Die detektierte Intensität des abgeschatteten Bereiches ist direkt proportional zur Belegungsdichte mit Markierungen wie beispielsweise Gold-Partikeln und dem Keimbildungszustandes der Partikel.

Bei Verwendung eines Niederschlags, der elektrisch leitfähig ist oder dessen Dielektrizitätskonstante sich von der Umgebung unterscheidet, ist der Nachweis der Reaktion bei einer alternativen Ausführungsform der vorliegenden Erfindung auch elektrisch möglich.

Die elektrischen Messungen können über Leitfähigkeitsmessungen mittels Mikroelektrodenarray-Anordungen oder über eine Anordnung von Mikrokapazitätssensoren oder über Potentialmessungen mittels Feld-Effekt-Transistoren-Arrays (FET-Arrays) erfolgen. Bei Leitfähigkeitsmessungen mittels Mikroelektroden wird die Änderung des elektrischen Widerstandes zwischen zwei Elektroden bei einer Abscheidungsreaktion verfolgt (E. Braun, Y. Eichen, U. Sivan, G. Ben-Yoseph, Nature, 775, vol 391, 1998). Bei Dielektrizitätsmessungen mit Mikrokapazitätssensoren wird die Änderung der Kapazität zweier zueinander angeordneten Elektroden gemessen (M. Madou, Fundamentals of Microfabrication, CRC Press, Boca Raton, 1997). Bei Potentialmessungen mittels FET-Arrays wird die Änderung des Potentials auf den Sensoroberflächen gemessen (M. Madou, Fundamentals of Microfabrication, CRC Press, Boca Raton, 1997).

Bei Verwendung eines Substrates, das radioaktiv ist, oder radioaktiv markiert ist, kann der Nachweis der Gegenwart eines Niederschlags auf einem Array-Element durch Autoradiographie, Fluorographie und/oder indirekte Autoradiographie erfolgen. So wird bei der Autoradiographie eine mit strahlendem Präzipitat bedeckte Fläche direkt mit einem Röntgenfilm in Kontakt gebracht. Bei der Fluorographie wird eine mit strahlendem Präzipitat bedeckte Fläche mit fluoreszierenden Chemikalien wie z.B. Natriumsylicylat überschichtet, die die radioaktive Strahlungsenergie in Fluoreszenz umwandeln. Bei der indirekten Autoradiographie mit Verstärkungsfolien (intensifier screens) wird eine mit β-strahlendem Präzipitat bedeckte Fläche auf eine Verstärkerfolie gelegt, die die Strahlung in blaues Licht umwandelt. (siehe F. Lottspeich, H. Zorbas, siehe oben). Auf Radioaktivität basierende Detektionsverfahren sind jedoch aufgrund der gesundheitlichen Risiken und der deswegen zu erfüllenden Sicherheitsvorschriften häufig unerwünscht.

Bei weiteren alternativen Ausführungsformen der vorliegenden Erfindung erfolgt der Nachweis der Gegenwart eines Niederschlags auf einem Array-Element durch Rasterelektronenmikroskopie, Elektronensonden-Mikroanalyse (EPMA), magnetooptische Kerr-Mikroskopie, magnetic force-Mikroskopie (MFM), atomic force-Mikroskopie (AFM), Messung des Mirage-Effekts, Rastertunnelmikroskopie (STM) und/oder Ultraschall-Reflexions-Tomographie.

Nahezu unabhängig von der Art des Substrates ist der Nachweis der Reaktion mittels SEM und/oder EPMA. Bei der Rasterelektronen-Mikroskopie (scanning electron microscopy (SEM)) tastet ein fokusierter Elektronenstrahl die Probe ab (J. Goldstein et al. Scanning Electron Microscopy and X-Ray Microanalysis, Plenum, New York, 1981). Bei der electron probe microanalysis (EPMA) werden die Sekundärprozesse, die durch einen fokusierten Elektronenstahl ausgelöst werden, zur ortsaufgelösten Analyse genutzt (J. Goldstein et al. Scanning Electron Microscopy and X-Ray Microanalysis, Plenum, New York, 1981).

Bei Verwendung eines Substrates, das magnetisch ist, oder mit Magnetpartikeln markiert ist, kann der Nachweis der Reaktion durch Magnetooptische Kerr-Mikroskopie oder MFM erfolgen. Bei der Magnetooptischen Kerr-Mikroskopie wird die Drehung der Polarisationsebene des Lichtes durch magnetische Felder (Kerr- und Faradayeffekt) ausgenutzt (A.Hubert, R.Schäfer, Magnetic Domains, Springer, 1998).

Die Änderung der optischen Dichte durch das Substrat auf der Oberfäche infolge der Reaktion kann mittels des Mirrage-Effektes detektiert werden. Beim Mirrage-Effekt wird die lokale Erwärmung einer Oberfläche durch Absorption eines gebündelten Laserstrahls über die damit verbundene Brechungsindexänderung gemessen. Durch das Abrastem der Oberfläche erhält man ein Bild von den lokalen Oberflächenabsorptionseigenschaften (A. Mandelis, Progress in Photothermal and Photoacoustic Science and Technology, Volume 1, Elsevier, New York 1992). Ein weiteres thermisches ortsaufgelöstes Verfahren zum Nachweis der Wechselwirkungsreah-tion durch das Substrat ist eine Array-Anordnung von Mikrothermophiles, die die Kristallisations- bzw. Abscheidungsenthalpien der Substratabscheidungen messen (J.M. Köhler, M. Zieren, Thermochimica acta, 25, vol 310, 1998).

Ebenfalls geeignet zum Nachweis der Reaktion mittels Substrat sind STM und AFM. Beim atomic force microscope (AFM) tastet eine Mikro- oder Nanospitze die Oberflächen ab, wodurch die Oberflächentopographie vermessen wird (E. Braun, Y. Eichen, U. Sivan, G. Ben-Yoseph, Nature, 775, vol 391, 1998). Das magnetic force microscope MFM detektiert über eine Nanospitze lokale magnetische Suszeptibilitätsunterschiede (A.Hubert, R.Schäfer, Magnetic Domains, Springer, 1998). Beim scanning tunneling microscop STM wird über eine Nanospitze der Tunnelstrom gemessen, um die Nanooberflächentopographie zu ermitteln (O. Marti, M. Amrein, STM and SFM in biology, Academic Press Inc., San Diego, 1993)

Exotischere Verfahren wie die Ultraschall-Reflexions-Tomographie können ebenfalls zur Anwendung kommen. Bei den Tomografien handelt es sich um Verfahren, bei denen scheibchenweise ein 3-dimensionales Bild zusammengetragen wird (F. Natterer, Mathematische Methoden der Computer-Tomographie, Westdt. Vlg., Wiesbaden, 1997). Im Falle der Ultraschall-Reflexions-Tomographie wird die Messung der Ultraschall - Reflexion ausgenutzt, um das Tomogramm zu erzeugen (V. Fleischer, F. Bergner, DGZfP NDT Conference Dresden 1997).

Bei einer speziellen Ausführungsform der vorliegenden Erfindung wird ein Verfahren zur Verfügung gestellt, daß die folgenden Schritte umfaßt:

- Detektion des zeitlichen Verlaufs der Niederschlagsbildung an den Array-Elementen durch Aufnahme von Bildern durch eine Kamera;
- Umwandlung der in den Bildern enthaltenen analogen Information in digitale Form;
- Berechnung einer virtuellen Signalintensität für jedes Array-Element anhand einer Kurvenfunktion, die die Niederschlagsbildung als Funktion der Zeit beschreibt;
- Umwandlung der virtuellen Signalintensitäten in ein künstliches Bild, das die virtuellen Signalintensitäten aller Array-Elemente darstellt.

Unter einem Bild wird im Rahmen der vorliegenden Erfindung eine Gruppe von Pixeln verstanden, die eine Veranschaulichung der gemessenen Signalintensitäten für einen Sonden-Array darstellt und die direkt beispielsweise an einen Bildschirm oder einen Drucker zur Aufzeichnung übermittelt werden kann.

Unter einem künstlichen Bild wird im Rahmen der vorliegenden Erfindung eine Gruppe von Pixeln verstanden, die eine Veranschaulichung von erfindungsgemäß bestimmten virtuellen Signalintensitäten für einen Sonden-Array darstellt und die ebenfalls beispielsweise auf einem Bildschirm oder einen Drucker dargestellt werden kann.

Ein weiterer Aspekt der vorliegenden Erfindung betrifft eine Vorrichtung zur Durchführung des vorstehend beschriebenen erfindungsgemäßen Verfahrens, umfassend:

a) ein Array-Substrat mit Sonden-Array,
b) eine Reaktionskammer,
c) eine Vorrichtung zur Detektion eines Niederschlags auf einem Array-Element, an dem eine Wechselwirkung zwischen Targets und Sonden stattgefunden hat, und
d) einen Computer, der programmiert ist, um:

- die von der Detektionsvorrichtung aufgenommenen Signalintensitäten zu sammeln;
- die Verarbeitung der sukzessive aufgenommenen Signalintensitäten derart zu gewährleisten, daß der zeitliche Verlauf der Niederschlagsbildung mit der Zeit auf einem Array-Element bestimmt wird und eine virtuelle Signalintensität anhand einer Kurvenfunktion bestimmt wird, die die Niederschlagsbildung als Funktion der Zeit beschreibt; und
- ggf. die Umwandlung der virtuellen Signalintensitäten in ein analoges Bild zu gewährleisten.

Die Detektionsvorrichtung ist vorzugsweise eine Kamera, insbesondere eine CCD-oder CMOS-Kamera oder ähnliche Kameras, die üblicherweise den gesamten Bereich des Sonden-Arrays aufzeichnet.

Wie bereits vorstehend erwähnt, läßt sich durch zeitaufgelöste Detektion während des Verstärkungsprozesses durch die Ablagerung eines Niederschlags, wie beispielsweise elementarem Silber auf als Kristallisationskeimen wirkenden Goldpartikeln, und die Berechnung der relativen Belegungsdichten aus dem Zeitverhalten nach dem erfindungsgemäßen Verfahren die dynamische Auflösung der Meßdaten selbst bei Verwendung einer 8 bit-Detektionstechnik extrem erhöhen. Der Aufbau einer hierzu nötigen Vorrichtung unterscheidet sich durch die mechanische Aufnahme einer Reaktionskammer und eine modifizierte Akquisitions-Software. Die Software ist dadurch gekennzeichnet, daß sie die Verarbeitung der sukzessive aufgenommenen Aufnahmen gestattet. Hierzu werden die über den einzelnen Sondenarray-Elementen ermittelten Grauwerte zu jedem Zeitpunkt ermittelt. Für alle Array-Elemente wird die virtuelle Siganlintensität in Abhängigkeit der Niederschlagbildung von der Zeit berechnet. Ausgehend von diesem Wert werden beispielsweise die Grauwerte der letzten Messung mit dem Produkt aus Rate und Meßdauer in Beziehung gesetzt und damit eine Spreizung des Meßbereichs erreicht. Somit ist selbst bei Verwendung von kostengünstigen 8 bit -Kameras eine ausgezeichnete Auflösung von schwachen neben starken Sonden/Target-Wechselwirkungen sowie eine genaue Quantifizierung der gebundenen Targets gewährleistet.

Bei einer bevorzugten Ausführungsform umfaßt die erfindungsgemäße Vorrichtung zusätzlich eine Lichtquelle, die besonders bevorzugt ausgewählt wird aus der Gruppe, bestehend aus einem Laser, einer Licht-emittierenden Diode (LED) und einer Hochdrucklampe.

Die Komponenten eines beispielhaften Aufbaus einer erfindungsgemäßen Vorrichtung zum optisch Nachweis einer Niederschlagsbildung bestehen aus einer leistungsschwachen (500mcd) Lichtquelle, z.B. einer LED, zur homogenen Beleuchtung und einem Detektor, z.B. einer CCD-Kamera. Aufgrund des Verstärkungseffekts über die katalytische Abscheidung des Substrates, insbesondere bei der Verwendung eines Gold/Silbersystems, sind die Änderungen der optischen Eigenschaften der Oberfläche derart ausgeprägt, daß ein einfacher Flachbettscanner, ein Diascanner oder ein vergleichbares Gerät zur Detektion des Niederschlags ausreicht.

Typische Detektionszeiten liegen deutlich unter 1s, während vergleichbare sensitive CCD-Systeme zur Detektion von Fluoreszenz etwa 10 s bis 80 s benötigen, so daß preiswerte Consumer-Kameras Verwendung finden können, deren Signalübertragung der Videonorm entspricht.

Das Miniaturisierungspotential eines solchen Aufbaus ist sehr hoch, das gesamte System kann als autonomes Handgerät für den Feldeinsatz konzipiert werden. Ferner kann die erfindungsgemäße Vorrichtung bei einer besonders bevorzugten Ausführungsform als hochintegrierte autonome Einheit realisiert werden. Damit sind hochsensive Anwendungen von Microarrays wie z.B. medizinische Diagnostik, Forensik, bakterielles Screening usw. auch unabhängig von medizinischen bzw. biologischen Laboratorien vom Laien schnell durchführbar.

Im folgenden ist eine potentielle Anwendung des erfindungsgemäßen Verfahrens bei der Gewebetypisierung in der Transplantationsmedizin beschrieben. Die Analyse der Struktur, Expression und Vererbung immunologisch relevanter Gene für Transplantation und Autoimmunität ist von besonderem Interesse, da sowohl für die spezifische Antigenerkennung (Histokompatibilitätsantigene, T-Lymphozytenrezeptor) als auch für die Effektormechanismen (Antikörper, Fc-Rezeptoren) hochgradig polymorphe Systeme existieren und diese komplexen genetischen Regulationsmechanismen unterliegen. Neben den schwachen tragen insbesondere die starken Transplantationsantigene zu beispielsweise einer Transplantatabstoßung bei. Diese starken Antigene werden als Haupthistokompatibilitätsantigene bezeichnet und sind genetisch innerhalb des Haupthistokompatibilitätskomplexes (MHC) kodiert. Der MHC, der bisher ausschließlich bei Vertebraten nachgewiesen wurde, wird beim Menschen als HLA (Humanes Leukozyten Antigen) bezeichnet. Der HLA-Komplex ist auf dem kurzen Arm des menschlichen Chromosoms 6 lokalisiert (6p21.1- 6p21.3) und umfaßt einen Bereich von ca. 3500 Kilobasen. Sehr grob lassen sich die HLA-Moleküle in zwei Klassen (Klasse I und Klasse II) unterteilen, die sich dann in weitere Subgruppierungen aufteilen. Die jeweiligen Genorte für die HLA-Produkte, welche in ihrer Summation für die entsprechenden immunologischen Eigenschaften des Organismus verantwortlich sind, treten durch die Prozesse der Vererbung in sehr zahlreichen Allelvariationen, deren bekannte Anzahl stetig zunimmt, auf.

Die Zahl der möglichen Alleltypisierungen des HLA-Systems, die an einem Organismus durch serologische und molekularbiologische Analyse exakt vorgenommen werden können, ermöglicht je nach medizinischer Relevanz der zu transplantierenden Zellspezies, eine beliebige Tiefe. Je tiefer die Typisierung und je größer die nachfolgende Übereinstimmung zwischen Spender und Empfänger ist, desto weniger sind Probleme wie Gewebeunverträglichkeiten und Transplantatabstoßung zu erwarten. Neben verschiedenen Transplantationen spielen aber auch Transfusionen, Krankheitsassoziationen und der forensische Bereich zur eindeutigen Personenidentifizierung eine Rolle.

In den Ausführungsbeispielen sind ein Prinzipnachweise für die Nachweisgrenze und Beispiele für Expressionsmonitoring dargestellt. Die verwendeten proprietären Prinzipien lassen sich jedoch auch für weitere Anwendungen benutzen. Neben den quantitativen Analysen, wie z.B. dem Expressionsmonitoring von Organismen lassen sich auch zahlreiche qualitative Analysen durchführen.

Z.B. treten die jeweiligen Genorte für die HLA-Produkte, welche in ihrer Summation für die entsprechenden immunologischen Eigenschaften des Organismus verantwortlich sind, durch die Prozesse der Vererbung in sehr zahlreichen Allelvariationen, deren bekannte Anzahl stetig zunimmt, auf. Die Zahl der möglichen Alleltypisierungen des HLA-Systems, die an einem Organismus durch serologische und molekularbiologische Analyse exakt vorgenommen werden können, ermöglicht je nach medizinischer Relevanz der zu transplantierenden Zellspezies, eine beliebige Tiefe. Je tiefer die Typisierung und je größer die nachfolgende Übereinstimmung zwischen Spender und Empfänger ist, desto weniger sind Probleme wie Gewebeunverträglichkeiten und Transplantatabstoßung zu erwarten. Seit der Entdeckung der MHC-Moleküle hat man zahlreiche Verfahren zur Charakterisierung des Polymorphismus dieser Moleküle und ihrer Gene entwickelt und eingesetzt. Eine grundsätzliche Differenz besteht zwischen biochemischen, zellulären und serologischen einerseits und den molekularbiologischen Techniken auf der anderen Seite. Erstere analysieren durch z.B. den Einsatz von spezifischen Antikörpern ausschließlich die Expressionsprodukte während die zweite Gruppe durch z.B. die Techniken der Hybridisierung und Amplifikation von Nukleinsäuren Sequenzunterschiede im Bereich kodierender und nichtkodierender Sequenzen nachweist (Bidwell J., 1994 Advances in DNA-based HLA-typing methods. Immunol Today, 15(7):303-7).Durch die beschriebene Erfindung wäre es gegeben, daß nach einer Isolierung, entsprechenden Markierung und gegebenenfalls Amplifikation für das Hervorheben diagnostisch relevanter Allelstrukturen zu einem Sequenzhintergrund der genomischen DNA eines Individuums, eine massiv-parallele Hybridisierung gegen ein Sondenarray (DNA-Chip) aller bekannter Allelvarianten mit dem Ziel der tiefst-möglichen HLA-Typisierung erfolgen würde. Dabei wird, durch den beschriebenen Hybridisierungsnachweis und die Signalverstärkung in Kombination mit einem einfachen Detektor verglichen mit anderen Verfahren, in einer minimalen Diagnostikzeit eine maximale genomische Typisierung unter Verwendung bekannter allelspezifischer Sonden in einem äußerst günstigen ökonomischen Rahmen zugänglich.

Ein weiteres mögliches Anwendungsfeld liegt im Pharma- und Diagnostikbereich. Bei der Metabolisierung von körpereigenen und körperfremden Substanzen (z.B. Pharmaka) im Organismus spielen eine Reihe von genetischen Polymorphismen, Mutationen, Deletionen etc. mit diesbezüglich funktionalen Effekten auf der Proteinebene eine wesentliche Rolle. Individuelle genotypische Verteilungen in diesen DNA-Sequenzen führen phänotypisch z.B. zu Korrelationen mit bestimmten Krankheitsbildern (so zum Beispiel bei dem Gen für p53 und dem Mamakarzinom sowie mitochondrialer Genvariationen der Gene D-loop, 16-S-rRNA, ND3-5, CytB, tRNATrp, tRNALeu und dem Lungen- und Blasenkarzinom (Fliss MS et. al (2000) Facile detection of mitochondrial DNA mutations in tumors and bodily fluids. Science. 17;287(5460):2017-9.) oder unterschiedlicher Wirkung von Xenobiotika auf den Organismus. Letzteres ist z. B. detailliert an Cytochrom P 450 Genen (CYP2D6, CYP2C19, CYP2A6, CYP2C9, CYP2E1), Gluthathion-S-Transferase Genen (GSTM1, GSTT1), dem N-Acetyltransferase Gen (NAT2), dem Apolipoprotein E Gen (ApoE) u.v.a.m. demonstriert worden. Die Ergebnisse all dieser Untersuchungen zusammengefasst erlauben die Erstellung eines Arrays mit DNA Sonden, welche die Sequenzunterschiede parallel detektieren. Im Zusammenhang mit dem beschriebenen Hybridisierungs- und Verstärkungsnachweis und einem einfachen optischen Aufbau sind so qualitative Genotypisierungen schnell und kostengünstig realisierbar. Diese sind als Muster sowohl beim Individuen-spezifischen Einsatz von Pharmaka als auch als Marker zur Personenidentifikation und bei der Diagnostik relevant.

Die folgenden Beispiele und Abbildungen dienen der Erläuterung der Erfindung und sollen nicht in einschränkender Weise ausgelegt werden.

**Beispiele**

Beispiel 1: Nachweis der Hybridisierung von Nukleinsäuren (quantitative Analyse)

Vorbereitung des Trägers

Auf mit einer epoxidierten Glasfläche der Größe 3 x 3 mm ("Sondenarray") wurde an einer definierten Stelle ("Arrayelement") ein aminomodifiziertes Oligonukleotid mit einer Länge von 20 Nukleotiden der Sequenz 5'-NH$_2$-CC-TCTGCAGACTACTATTAC-3' kovalent immobilisiert. Dazu wurden 0,1 μl einer 5 μM Lösung des Oligonukleotides in 0,5 M Phosphatpuffer auf der Glasfläche abgelegt und schließlich bei 37°C eingetrocknet. Die kovalente Verknüpfung der abgelegten Oligonukleotide mit den Epoxid-Gruppen auf der Glasoberfläche erfolgte durch 30-minütiges Backen der Sondenarrays bei 60°C. Anschließend wurden die Sondenarrays kräftig mit destilliertem Wasser gespült und danach 30 min in 100 mM KCl gewaschen. Nach weiterem kurzen Spülen in 100 mM KCl und anschließend destilliertem Wasser wurden die Sondenarrays 10min bei 37°C getrocknet.

Hybridisierung des komplementären Oligonukleotides

Für die Hybridisierung wurde ein komplementäres Biotin-markiertes 20 bp langes Oligonukleotid der Sequenz 5'-Bio-GTAATAGTAGTCTGCAGAGG-3' eingesetzt. Die Reaktion wurde in den folgenden Konzentrationsabstufungen in einem Puffer (0,25 M NaPO$_4$, 4,5% SDS, 1 mM EDTA in 1xSSC) in einem Gesamtvolumen von je 50 μl aufgenommen:

10 nM, 1 nM, 100 pM, 10 pM, 1 pM, 100 fM, 10 fM, 1 fM.

Je Konzentrationsstufe wurde ein vorbereitetes Sondenarray in die Hybridisierungslösung gegeben. Der so erhaltene Hybridisierungsansatz wurde 5 min bei 95°C und danach 60 min bei 50°C inkubiert. Anschließend wurden die Sondenarrays unter Schütteln je 10 min in 2xSSC+0,2%SDS, 2xSSC und 0,2xSSC (Maniatis et al., 1989) gewaschen und mit Druckluft trockengeblasen.

Nachweis der Hybridisierung

Ein Streptavidin-Goldkonjugat wurde in einer 1:50 Verdünnung in 6xSSPE(52,5 g NaCl, 26,4 g $NaH_2PO_4$ x $H_2O$, 2,22 g NaOH aufgefüllt mit $H_2O$ auf 1 Gesamtvolumen) + 0,005% Triton-Lösung auf den Sondenarray gegeben und 15 min bei 30°C inkubiert. Anschließend wurden die Sondenarrays unter Schütteln je 10 min in 2xSSC(17,5 g NaCl, 8,8g NaCitrat in 1 1 $H_2O$, mit 10 N NaOH auf pH 7,0 eingestellt) + 0,2% SDS (Natriumdodecylsulfat), 2xSSC und 0,2xSSC gewaschen und mit Druckluft trockengeblasen. Alternativ zu dem Goldkonjugat des Streptavidins wurden auch direkt mit Goldpartikeln modifizierte Targets eingesetzt.

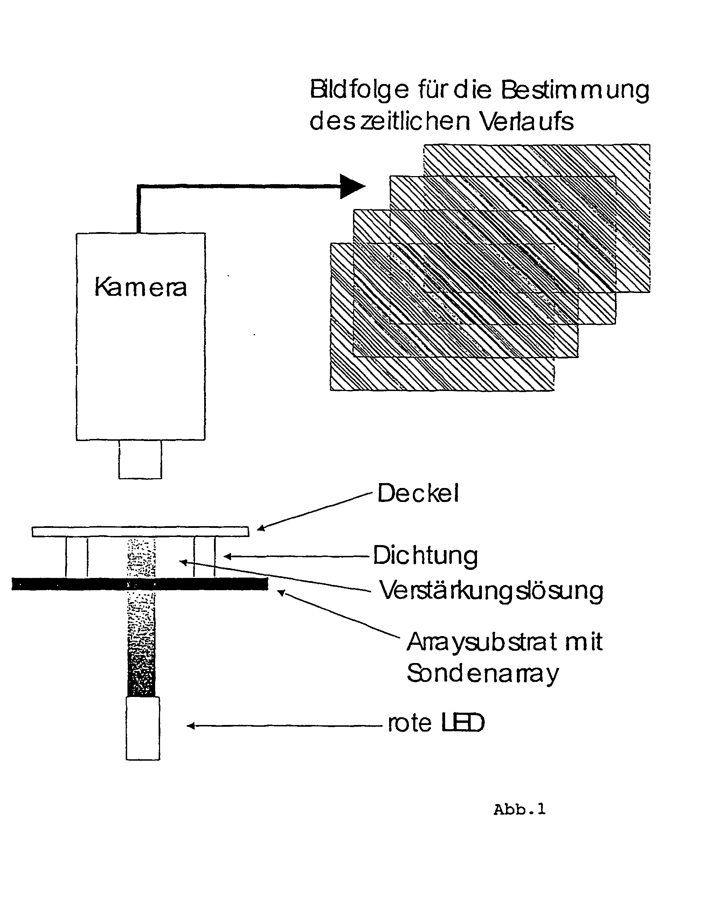

Die nachfolgende Verstärkung der nunmehr auf dem Sondenarray immobilisierten Goldpartikel erfolgte mit 0,1% Silbernitratlösung in 3% Natriumkarbonat und 0.02% Formaldehydlösung. Das Gemisch wurde kurz vor der Reaktion frisch angesetzt. Innerhalb der 15-minütigen Inkubation wurde bei 22°C und rotem Licht die Reaktion beobachtet und kontinuierlich mit der in Abbildung 1 dargestellten Vorrichtung aufgezeichnet.

Die Nachweisgrenze wurde mit < 10 pM gefunden.

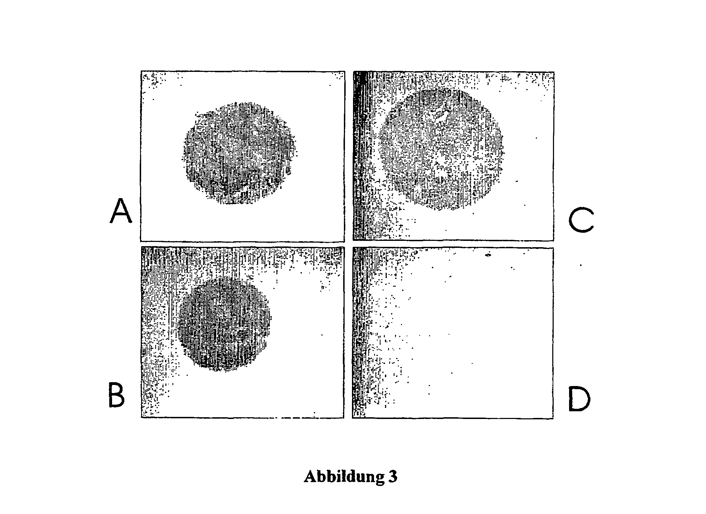

In Abbildung 3 sind die Ergebnisse der Hybridisierung dargestellt:

A - Hybridisierung des in einer Konzentration von 10 nM vorliegenden Targets,
B - Hybridisierung des in einer Konzentration von 1 nM vorliegenden Targets,
C - Hybridisierung des in einer Konzentration von 100 pM vorliegenden Targets und
D - Hybridisierung des in einer Konzentration von 10 pM vorliegenden Targets.

Beispiel 2: Prinzipnachweis zum Einsatz des Verfahrens im Expression Profiling - Nachweis der Hybridisierung von genomischer RNA aus *Corynebacterium glutamicum* gegen ein Sondenarray von 356 Sonden

DNA-Arrays werden häufig dazu verwendet, den globalen physiologischen Zustand von Zellen zu messen (expression profiling). Dazu wird RNA aus den entsprechenden Zellen isoliert, mit einer geeigneten Methode markiert und gegen ein Sonden-Array mit komplementären Sonden hybridisiert. Im folgenden Anwendungsbeispiel wurde das erfindungsgemäße Verfahren zur Detektion von zellulären mRNAs aus *Corynebacterium glutamicum* eingesetzt.

Herstellung der Sonden-Arrays

Auf mit einem standardisierten und epoxidierten mikroskopischen Objektträger der Firma CLONDIAG Chip Technologies (Jena, Deutschland), der als Arraysubstrat dient, wurde ein Sondenarray von 356 verschiedenen aminomodifizierten Oligonukleotiden von 25 bzw. 30 Basen Länge und c-DNAs unterschiedlicher Länge erzeugt. Alle Oligonukleotide waren komplementär zu Teilsequenzen der *aceA*- und *icd*-Gene. Die Sondenarrays wurden durch Arraying mit dem Micro-Grid I Arrayer der Firma Biorobotics Ltd. (Großbritannien) nach Herstellerangaben erzeugt, indem die aminomodifizierten DNAs in einer Endkonzentration von 5 μM in 0,5 M Phosphatpuffer auf den Objektträger aufgebracht und schließlich eingetrocknet wurden. Die kovalente Verknüpfung der abgelegten Oligonukleotide mit den Epoxid-Gruppen auf der Glasoberfläche erfolgte durch 30-minütiges Backen der Objektträger bei 60°C. Anschließend wurden die Slides kräftig mit destilliertem Wasser gespült und danach 30 min in 100 mM KCl gewaschen. Nach weiterem kurzen Spülen zunächst in 100 mM KCl und anschließend in destilliertem Wasser wurden die Sondenarrays 10 min bei 37°C getrocknet.

Vorbereitung der Gesamt-RNA aus *Corynebacterium glutamicum*

Gesamt-RNA aus *Corynebacterium glutamicum* wurde mittels des Fast RNA Kits (Fa. Bio 101) nach Herstellerangaben gewonnen. 50 μg RNA wurden mittels Biotin Chem Link (Boehringer Mannheim, Deutschland) nach Herstellerangaben bei 85°C für 30 Minuten biotinyliert. Die RNA wurde anschließend über Microcon-30-Säulen (Fa. Millipore) nach Herstellerangaben eingeengt und anschließend mehrmals mit entionisiertem, RNase-freiem Wasser gewaschen. Das Eluat wurde im Vakuum auf 5 μl eingeengt.

Hybridisierung der RNA

Die biotinylierte RNA wurde in 100 µl Hybridisierungspuffer (0,25 M NaPO$_4$, 4,5% SDS, 1 mM EDTA in 1xSSC) aufgenommen und für 3 Minuten bei 65°C denaturiert. Die mit DNA belegte Fläche des Objektträgers wurde mit einer Hybridisierungskammer (Hybrislip, Sigma, Deisenhofen, Deutschland) gedeckelt. Das Slide wurde auf einem Thermoschüttler mit Mikrotiterplattenaufsatz (Eppendorf, Hamburg, Deutschland) auf 50°C vortemperiert. Anschließend wurde die denaturierte Hybridisierungslösung eingefüllt und die Hybridisierungskammer nach Herstellerangaben verschlossen. Die Inkubation wurde für 60 min bei 50°C fortgesetzt. Anschließend wurde die Hybridisierungslösung abgenommen, die Hybridisierungskammer entfernt und die Slides unter Schütteln 10 min bei 30°C in 2xSSC+0,2%SDS und jeweils 10 Minuten bei Raumtemperatur in 2xSSC und 0,2xSSC (Maniatis et al., 1989) gewaschen und mit Druckluft trockengeblasen.

Nachweis der Hybridisierung

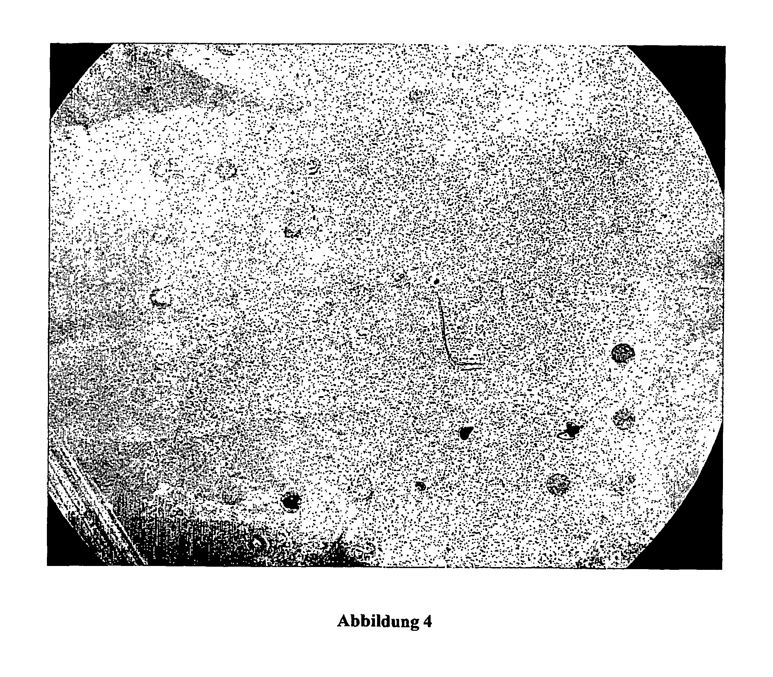

Ein Streptavidin-Goldkonjugat (EM.STP5, Fa. British BioCell International) wurde in einer 1:50 Verdünnung in 6xSSPE+0,005%Triton (Maniatis et al., 1989) auf das Slide gegeben und 15 min bei 30°C inkubiert. Anschließend wurden die Sondenarrays unter Schütteln je 10 min in 2xSSC+0,2%SDS, 2xSSC und 0,2xSSC gewaschen und mit Druckluft getrocknet.
Die nachfolgende Verstärkung der nunmehr auf dem Sondenarray immobilisierten Goldpartikel erfolgte mit dem LM/ EM Silver Enhancing Kit (SEKL15, British BioCell International). Gemäß den Angaben der Herstellers wurden je 2 Tropfen der Initiator- und der Enhancer-Lösung vermischt und davon je 15µl auf die Sondenarrayoberfläche pipettiert. Innerhalb der 15-minütigen Inkubation wurde bei 22°C und rotem Licht die Reaktion beobachtet und kontinuierlich mit der in Abbildung 1 dargestellten Vorrichtung aufgezeichnet. Das resultierende Muster kann auch nach 15minütiger Inkubation abschließend betrachtet werden (siehe Abbildung 4).

Beispiel 3: Nachweis und Spezifität der Hybridisierung von Nukleinsäuren

Herstellung der Sondenarrays

Auf einer epoxidierten 3D-Objektträger (75 mm x 25 mm)-Glasoberfläche (Fa. Elipsa) wurden mit einem Micro-Grid II-Arrayer (Fa. BioRobotics) 16 aminomodifizierte Oligonukleotide (Sonden) mit einer Länge von jeweils 16 Nukleotiden an definierten Stellen abgelegt und kovalent immobilisiert (Arrayelemente). Die Sequenzen der Oligonukleotide waren folgende (jeweils mit einer 3'-NH$_2$-Modifikation):

```
1:    3'-    ATG  GCG  TTT  AGA  ACC  C      –5'


2:    3'-    ATG  CCG  TAT  GGA  ATC  C      –5'


3:    3'-    ATG  TCG  TGT  CGA  AAC  C      –5'


4:    3'-    ATG  ACG  TCT  TGA  AGC  C      –5'


5:    3'-    ACG  GCA  TTT  AGT  ACC  G      –5'
```

6:    3'-    ACG  CCA  TAT  GGT  ATC  G    –5'

7:    3'-    ACG  TCA  TGT  CGT  AAC  G    –5'

8:    3'-    ACG  ACA  TCT  TGT  AGC  G    –5'

9:    3'-    AGG  GCT  TTT  AGC  ACC  A    –5'

10:    3'-    AGG  CCT  TAT  GGC  ATC  A    –5'

11:    3'-    AGG  TCT  TGT  CGC  AAC  A    –5'

12:    3'-    AGG  ACT  TCT  TGC  AGC  A    –5'

13:    3'-    AAG  GCC  TTT  AGG  ACC  T    –5'

14:    3'-    AAG  CCC  TAT  GGG  ATC  T    –5'

15:    3'-    AAG  ACC  TCT  TGG  AGC  T    –5'

16:    3'-    AAG  TCC  TGT  CGG  AAC  T    –5'

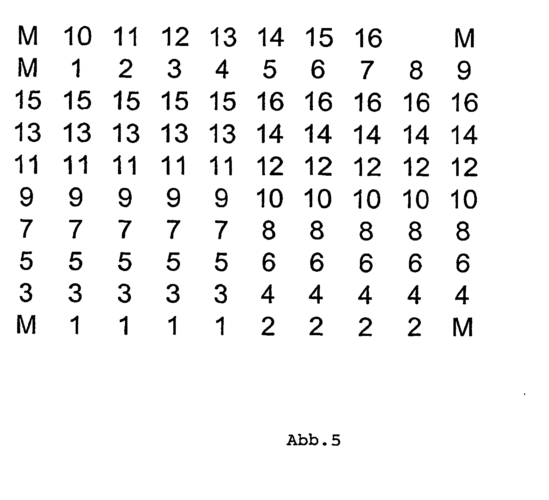

Ein einzelnes komplettes (quadratisches) Sondenarray auf der Objektträger-Oberfläche bestand aus insgesamt 10 x 10 = 100 abgelegten Sonden. Jede der 16 Oligonukleotid-Sonden wurde dabei in mindestens 5-facher Wiederholung auf dem Sondenarray abgelegt (Array-Aufbau: siehe Abb. 5). Die Sonden hatten einen Abstand von 0,2 mm, das gesamte Sondenarray bedeckte eine Fläche von 2 mm x 2 mm. Insgesamt konnten auf diese Weise pro Objektträger über 100 identische Sondenarrays erzeugt werden.

Das Ablegen der Sonden erfolgte aus einer jeweils 10 µM-Lösung der Oligonukleotide in 0,1M Phosphatpuffer/ 5%-Natriumsulfat. Nach dem Ablegen und Eintrocknen wurden die Sonden durch 30-minütiges Backen bei 60 °C kovalent mit den Epoxid-Gruppen auf der Glasoberfläche verknüpft. Anschließend erfolgte ein Wasch- und Blockierungsprozess der Objektträger in folgender Reihenfolge:

5 min in 600 ml $H_2O$ bidest + 600 µl Triton x100

2 x 2 min in 600 ml $H_2O$ bidest + 60 µl HCL (konz.)

30 min in 100 mM KCl-Lösung
1 min in $H_2O$ bidest spülen

15 min bei 50°C in Glasschale in 75 ml H$_2$O bidest + 25 ml Ethylenglykol +20 µl HCl (konz.) inkubieren

1 min in H$_2$O bidest spülen

mit Druckluft trocknen

Nach Beendigung der Waschschritte und Trocknen der Objektträger wurden diese in 3,25 mm x 3,25 mm große Glasstücke (nachfolgend "Chips" genannt) zerschnitten. Auf jedem der Chips befand sich genau ein Sondenarray der Größe 2 mm x 2 mm.

Hybridsierung der Sondenarrays

Für die Hybridisierung der Sondenarrays standen für alle 16 Oligonukleotid-Sonden die komplementären Biotin-markierten 16 bp langen Oligonukleotide als Targets zur Verfügung. Als Beispiel sei hier nur das zur Oligonukleotid-Sonde 9 komplementäre Target "9b" mit folgender Sequenz angeführt:

$$\text{5'-Biotin} \quad \text{TCC} \quad \text{CGA} \quad \text{AAA} \quad \text{TCG} \quad \text{TGG} \quad \text{T} \quad \text{-3'}$$

Die Hybridisierungs-Reaktion wurde in 6x SSPE-Puffer (52,59 g NaCl, 8,28 g NaH$_2$PO$_4$ x H$_2$O, 2,22 g EDTA x 2H$_2$O in 1 1 H$_2$O bidest, auf pH 7,4 mit NaOH eingestellt)/0,1 % SDS in einem Gesamtvolumen von 70 µl mit den Target-Konzentrationsstufen 100 nM, 10 nM, 1nM, 100 pM, 10 pM, 1 pM durchgefiihrt. Je Konzentrationsstufe wurde ein Chip mit dem Sondenarray in die Hybridisierungslösung gegeben, 5 min bei 95 °C erhitzt, dann 60 min bei 30 °C unter Schütteln inkubiert. Danach wurde der Chip in ein neues Reaktionsgefäß mit 500 µl Hybridisierungspuffer (ohne Target) überführt und 10 min bei 55 °C bzw. 60 °C unter Schütteln gewaschen. Anschließend wurden die Chips dann noch je 10 min in 2x SSC/0,2 % SDS (500 µl bei 30 °C), 2x SSC (500 µl bei 20 °C) und 0,2x SSC (500 µl bei 20 °C) unter Schütteln gewaschen und dann getrocknet (Eppendorf-Concentrator).

Nachweis der Hybridisierung (Konjugation und Silberanfärbung)

Die hybridisierten und getrockneten Chips wurden in ein neues Reaktionsgefäß mit µl einer Strepiavidin-Goldkonjugat-Lösung in 6 x SSPE/0,1 % SDS-Puffer überführt und dort 15 min bei 30 °C inkubiert. Als Streptavidin-Goldkonjugat wurden Goldpartikel der Größe 5 nm verwendet (British Biocell International, EM.STP5). Das Konjugat lag im Versuch in einer Konzentration von 500 pg Streptavidin/µl vor.

Nach der Konjugation wurden die Chips je 10 min in 2x SSC/0,2 % SDS (500 µl bei 30 °C), 2x SSC (500 µl bei 20 °C) und 0,2x SSC (500 µl bei 20 °C) unter Schütteln gewaschen und dann getrocknet (Eppendorf-Concentrator).

Alternativ zu dieser Vorgehensweise wurde die Streptavidin-Goldkonjugat-Kopplung auch direkt in der Hybridisierungslösung durchgeführt. Hierzu wurde nach der 60-minütigen Hybridisierung das Streptavidin-Goldkonjugat direkt in die Hybridisierungslösung hinzugegeben und dann weitere 15 min bei 30 °C inkubiert. Danach wurde der Chip in ein neues Reaktionsgefäß mit 500 µl Hybridisierungspuffer (ohne Target) überführt und 10 min bei 55 °C bzw. 60 °C unter Schütteln gewaschen. Anschließend wurden die Chips dann noch je 10 min in 2x SSC/0,2 % SDS (500 µl bei 30 °C), 2x SSC (500 µl bei 20 °C) und 0,2x SSC (500 µl bei 20 °C) unter Schütteln gewaschen und dann getrocknet (Eppendorf-Concentrator).

Zur Silberverstärkung wurden die Chips in ein neues Reaktionsgefäß überführt und unter Schütteln für 10 min bei 25 °C in ca. 100 µl einer Silberentwicklungslösung (British Biocell International, SEKL15) inkubiert. Die Inkubationslösung wurde durch Mischen von je einem Tropfen Initiator- und Enhancer-Lösung hergestellt. Anschließend wurde der Chip 2 min in 500 µl 0,2 x SSC gewaschen und getrocknet (Eppendorf-Concentrator).

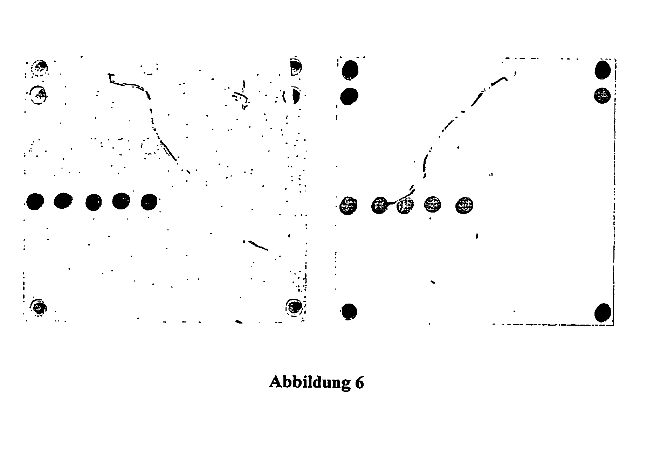

Die Ergebnisse zweier - exemplarisch ausgewählter - Hybridisierungen und deren Nachweis sind in Abbildung 6a und 6b dargestellt (Durchlicht-Aufnahmen).

Beispiel 4: Nachweis und Spezifität der Hybridisierung von Nukleinsäuren

Für das CFTR-Gen sind mehr als 800 Mutationen in der Literatur beschrieben, die zum Krankheitsbild der Cystischen Fibrose führen können. Im CFTR-Gen treten drei Typen von Mutationen auf:

- Basenaustausch (hier: Punktmutationen)
- Insertionen

- Deletionen

Für alle drei Mutationsformen sollte getestet werden, ob der jeweilige Wildtyp (pm) von der Mutation (mm) mittels Silberverstärkungsdetektion unterscheidbar ist. Sonden und Targets wurden von der Fa. Ogham (Münster, Deutschland) bereitgestellt.

Herstellung der Sondenarrays

Auf einer epoxidierten 3D-Objektträger (75 mm x 25 mm)-Glasoberfläche (Fa. Elipsa) wurden mit einem Micro-Grid II-Arrayer (Fa. BioRobotics) 10 aminomodifizierte Oligonukleotide (Sonden) mit einer Länge von 16 bis 22 Nukleotiden an definierten Stellen abgelegt und kovalent immobilisiert (Arrayelemente). Die 10 Sonden gliedern sich in 5 Paare, wobei die erste jeweils den Wildtyp und die zweite die Mutation darstellt. Das Sondenpaar 1 und 2 stellt eine Punktmutation dar, das Paar 3 und 4 eine Deletion und die Paare 5/6, 7/8, 9/10 Insertionen. Die Sequenzen der Oligonukleotide waren folgende:

Sequenz in 5' - 3'-Richtung mit 3'-NH$_2$-Modifikation:

| | | |
|---|---|---|
| 1: | GATCTTCGCCTTACTG | pm |
| 2: | GATCTTCACCTTACTG | mm |
| 3: | GAAACACCAAAGATGATA | pm |
| 4: | GAAACACC   GATGATA | mm |
| 5: | CTTCTAATTA TTTGGTATGT | pm |
| 6: | CTTCTAATTATTTGGTATGT | mm |
| 7: | GAGTTCTTCTAATTA TTTGG | pm |
| 8: | GAGTTCTTCTAATTATTTGG | mm |
| 9: | TTTTAGAGTTCTTCTAATTA T | pm |

10: TTTTAGAGTTCTTCTAATTATT     mm

Das Sondenpaar 3 (Wildtyp) und 4 (Deletion) beinhaltet die häufigste Mutation (70% aller Fälle), die für cystische Fibrose kodiert.

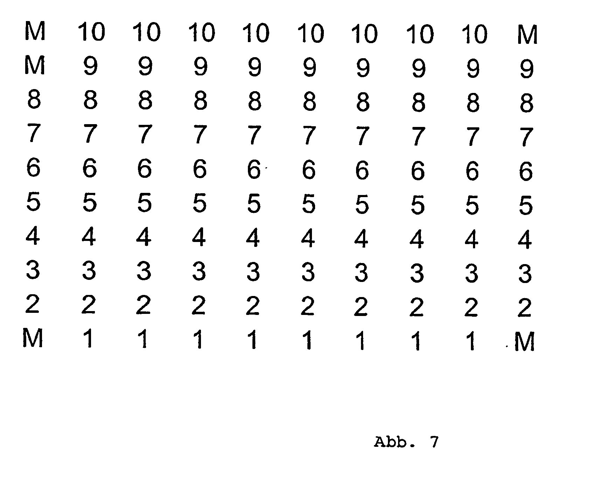

Ein einzelnes komplettes (quadratisches) Sondenarray auf der Objektträger-Oberfläche bestand aus insgesamt 10 x 10 =100 abgelegten Sonden. Jede der 10 Oligonukleotid-Sonden wurde dabei in 8- bis 10-facher Wiederholung auf dem Sondenarray abgelegt (Array-Aufbau: siehe Abbildung 7). Die Sonden hatten einen Abstand von 0,2 mm, das gesamte Sondenarray bedeckte eine Fläche von 2 mm x 2 mm. Insgesamt konnten auf diese Weise pro Objektträger über 100 identische Sondenarrays erzeugt werden.

Das Ablegen der Sonden erfolgte aus einer jeweils 10 µM-Lösung der Oligonukleotide in 0,1 M Phosphatpuffer/5%-Natriumsulfat. Nach dem Ablegen und Eintrocknen wurden die Sonden durch 30-minütiges Backen bei 60 °C kovalent mit den Epoxid-Gruppen auf der Glasoberfläche verknüpft. Anschließend erfolgte ein Wasch- und Blockierungsprozess der Objektträger in folgender Reihenfolge:

5 min in 600 ml $H_2O$ bidest + 600 µl Triton x100

2 x 2 min in 600 ml $H_2O$ bidest + 60 µl HCL (konz.)

30 min in 1o0 mM KCl-Lösung
1 min in $H_2O$ bidest spülen

15 min bei 50°C in Glasschale in 75 ml $H_2O$ bidest + 25 ml Ethylenglykol +20 µl HCl (konz.) inkubieren

1 min in $H_2O$ bidest spülen
mit Druckluft trocknen

Nach Beendigung der Waschschritte und Trocknen der Objektträger wurden diese in 3,25 mm x 3,25 mm große Glasstücke (nachfolgend "Chips" genannt) zerschnitten. Auf jedem der Chips befand sich genau ein Sondenarray der Größe 2 mm x 2 mm.

Hybridisierung und Konjugation der Sondenarrays

Für die Hybridisierung der Sondenarrays standen für die 10 Oligonukleotid-Sonden 3 zu den Perfect match (pm)-Sonden komplementäre Biotin-markierte Targets zur Verfügung. Dabei deckte Target 1 das Sondenpaar 1 und 2, Target 2 das Paar 3 und 4 und Target 3 die Sondenpaare 5/6, 7/8 und 9/10 ab. Die Sequenzen der Targets lauteten:

Target 1:     5'-Biotin- CTCAGTAAGGCGAAGATCTT-3'

Target 2:     5'-Biotin- AATATCATCTTTGGTGTTTCCT-3'

Target 3:     5'-Biotin- GAACATACCAAATAATTAGAAGAACTCTAAAACA-3'

Die Hybridisierungs-Reaktion wurde in 6 x SSPE-Puffer (52,59 g NaCl, 8,28 g $NaH_2PO_4$ x $H_2O$, 2,22 g EDTA x $2H_2O$ in 11 $H_2O$ bidest, auf pH 7,4 mit NaOH eingestellt)/0,1 % SDS in einem Gesamtvolumen von 70 µl mit verschiedenen Target-Konzentrationsstufen durchgeführt. Hierbei wurde ein Chip mit dem Sondenarray in die Hybridisierungslösung gegeben, 5 min bei 95 °C erhitzt, dann 60 min bei 30 °C unter Schütteln inkubiert.

Nach der 60-minütigen Hybridisierung wurde ein Streptavidin-Goldkonjugat direkt in die Hybridisierungslösung hinzugegeben und dann weitere 15 min bei 30 °C inkubiert. Als Streptavidin-Goldkonjugat wurden Goldpartikel der Größe 5 nm verwendet (British Biocell International, EM.STP5). Das Konjugat lag im Versuch in einer Konzentration von 500 pg Streptavidin/µl vor.

Nach der Hybridisierung und Konjugation wurde der Chip in ein neues Reaktionsgefäß mit 500 µl Hybridisierungspuffer (ohne Target) überführt und 10 min bei 55 °C unter Schütteln gewaschen. Anschließend wurden die Chips dann noch je 10 min in 2x SSC/0,2 % SDS (500 µl bei 30 °C), 2x SSC (500 µl bei 20 °C) und 0,2x SSC (500 µl bei 20 °C) unter Schütteln gewaschen und dann getrocknet (Eppendorf-Concentrator).

Silberverstärkung

Zur Silberverstärkung wurden die Chips in ein neues Reaktionsgefäß überführt und unter Schütteln für 10 min bei 25 °C in ca. 100 µl einer Silberentwicklungslösung (British Biocell International, SEKL15) inkubiert. Die Inkubationslösung wurde durch Mischen von je einem Tropfen Initiator- und Enhancer-Lösung hergestellt. Anschließend wurde der Chip 2 min in 500 µl 0,2x SSC gewaschen und getrocknet (Eppendorf-Concentrator).

Die Ergebnisse von drei Hybridisierungen und deren Nachweis sind in den Abb. 8, 9 und 10 dargestellt (Durchlicht-Aufnahmen). Während sowohl bei der Punktmutation (Abb. 8) und der Deletion (Abb. 9) eine deutliche Diskriminierung zwischen Wildtyp (pm) und Mutation (mm) erkennbar ist, trifft dies für die Insertion (Abb. 10) nicht zu. Hier zeigt bei dem Sondenpaar 9/10 der mm (Sonde 10) sogar ein stärkeres Signal als der Wildtyp (Sonde 9). Die Nachweisgrenze der Hybridisierung in diesem Versuchsaufbau lag bei einer Targetkonzentration von 10 pM.

Beispiel 5: Prinzipnachweis zum Einsatz des Verfahrens mit einem Oligonukleotid-Gold-Konjugat

Herstellung der Sondenarrays

Auf einer epoxidierten 3D-Objektträger (75 mm x 25 mm)-Glasoberfläche (Fa. Elipsa) wurden mit einem Micro-Grid II-Arrayer (Fa. BioRobotics) 16 aminomodifizierte Oligonukleotide (Sonden) mit einer Länge von jeweils 16 Nukleotiden an definierten Stellen abgelegt und kovalent immobilisiert (Arrayelemente). Die Sequenzen der Oligonukleotide (jeweils mit 3'-NH$_2$-Modifikation) waren wie folgt:

1: 3'- ATG GCG TTT AGA ACC C –5'

2: 3'- ATG CCG TAT GGA ATC C –5'

3: 3'- ATG TCG TGT CGA AAC C –5'

4: 3'- ATG ACG TCT TGA AGC C –5'

5: 3'- ACG GCA TTT AGT ACC G –5'

6: 3'- ACG CCA TAT GGT ATC G –5'

7:    3'-    ACG  TCA  TGT  CGT  AAC  G    –5'

8:    3'-    ACG  ACA  TCT  TGT  AGC  G    –5'

9:    3'-    AGG  GCT  TTT  AGC  ACC  A    –5'

10:   3'-    AGG  CCT  TAT  GGC  ATC  A    –5'

11:   3'-    AGG  TCT  TGT  CGC  AAC  A    –5'

12:   3'-    AGG  ACT  TCT  TGC  AGC  A    –5'

13:   3'-    AAG  GCC  TTT  AGG  ACC  T    –5'

14:   3'-    AAG  CCC  TAT  GGG  ATC  T    –5'

15:   3'-    AAG  ACC  TCT  TGG  AGC  T    –5'

16:   3'-    AAG  TCC  TGT  CGG  AAC  T    –5'

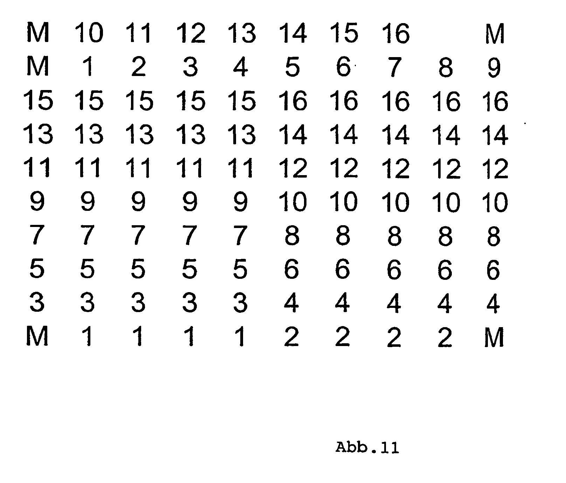

Ein einzelnes komplettes (quadratisches) Sondenarray auf der Objektträger-Oberfläche bestand aus insgesamt 10 x 10 =100 abgelegten Sonden. Jede der 16 Oligonukleotid-Sonden wurde dabei in mindestens 5-facher Wiederholung auf dem Sondenarray abgelegt (Array-Aufbau: siehe Abbildung 11). Die Sonden hatten einen Abstand von 0,2 mm, das gesamte Sondenarray bedeckte eine Fläche von 2 mm x 2 mm. Insgesamt konnten auf diese Weise pro Objektträger über 100 identische Sondenarrays erzeugt werden.

Das Ablegen der Sonden erfolgte aus einer jeweils 10 µM-Lösung der Oligonukleotide in 0,1 M Phosphatpuffer/5%-Natriumsulfat. Nach dem Ablegen und Eintrocknen wurden die Sonden durch 30-minütiges Backen bei 60°C kovalent mit den Epoxid-Gruppen auf der Glasoberfläche verknüpft. Anschließend erfolgte ein Wasch- und Blockierungsprozess der Objektträger in folgender Reihenfolge:

5 min in 600 ml $H_2O$ bidest + 600 µl Triton x100

2 x 2 min in 600 ml $H_2O$ bidest + 60 µl HCL (konz.)

30 min in 1o0 mM KCl-Lösung
1 min in $H_2O$ bidest spülen

15 min bei 50°C in Glasschale in 75 ml $H_2O$ bidest + 25 ml Ethylenglykol +20 µl HCl (konz.) inkubieren

1 min in $H_2O$ bidest spülen

mit Druckluft trocknen

Nach Beendigung der Waschschritte und Trocknen der Objektträger wurden diese in 3,25 mm x 3,25 mm große Glasstücke (nachfolgend "Chips" genannt) zerschnitten. Auf jedem der Chips befand sich genau ein Sondenarray der Größe 2 mm x 2 mm.

Herstellung des Oligonukleotid-Gold-Konjugates

Zur Herstellung eines Oligonukleotid-Gold-Konjugates wurden 5,4 nmol eines modifizierten Oligonukleotides (in 80 μl $H_2O$ bidest gelöst) mit der Sequenz 5'-Thiol-TTTTTTTTTTTTTTTTTTTT-3'("T20-Thiol") mit 6 nmol Monomaleimido-Nanogold (Fa. Nanoprobes) gemischt und 24 h bei 4 °C inkubiert. Das Nanogold wurde in 20 μl Isopropanol und 180 μl $H_2O$ bidest gelöst.

Hybridsierung und Konjugation der Sondenarrays

Für die Hybridisierung der Sondenarrays standen für alle 16 Oligonukleotid-Sonden die komplementären 36 bp langen Oligonukleotide als Targets zur Verfügung, die als Modifikation am 3'-Ende einen polyA-Schwanz aufwiesen. Als Beispiel sei hier nur das zur Oligonukleotid-Sonde 9 komplementäre Target "9c" mit folgender Sequenz angeführt:

**5'-TCCCGAAAATCGTGGTAAAAAAAAAAAAAAAAAAAA–3'**

Die Hybridisierungs-Reaktion wurde in 6x SSPE-Puffer (52,59 g NaCl, 8,28 g $NaH_2PO_4$ x $H_2O$, 2,22 g EDTA x $2H_2O$ in 1 l $H_2O$ bidest, auf pH 7,4 mit NaOH eingestellt)/0,1 % SDS in einem Gesamtvolumen von 70 μl mit verschiedenen Target-Konzentrationsstufen durchgeführt. Je Konzentrationsstufe wurde ein Chip mit dem Sondenarray in die Hybridisierungslösung gegeben, 5 min bei 95 °C erhitzt, dann 60 min bei 30 °C unter Schütteln inkubiert. Danach wurde das T20-Nanogold-Konjugat in verschiedenen Verdünnungsstufen in die Hybridisierungslösung hinzugegeben und weitere 30 min bei 30 °C inkubiert.

Anschließend wurde der Chip in ein neues Reaktionsgefäß mit 500 μl Hybridisierungspuffer (ohne Target und T20-Nanogold) überführt und 10 min bei 55 °C bzw. 60 °C unter Schütteln gewaschen. Abschließend wurden die Chips dann noch je 10 min in 2x SSC/0,2 % SDS (500 μl bei 30 °C), 2x SSC (500 μl bei 20 °C) und 0,2x SSC (500 μl bei 20 °C) unter Schütteln gewaschen und dann getrocknet (Eppendorf-Concentrator).

Silberverstärkung

Zur Silberverstärkung wurden die Chips in ein neues Reaktionsgefäß überführt und unter Schütteln für 10 min bei 25 °C in ca. 100 μl einer Silberentwicklungslösung (British Biocell International, SEKL15) inkubiert. Die Inkubationslösung wurde durch Mischen von je einem Tropfen Initiator- und Enhancer-Lösung hergestellt. Anschließend wurde der Chip 2 min in 500 μl 0,2x SSC gewaschen und getrocknet (Eppendorf-Concentrator).

Beispiel 6: Nachweis und Spezifität der Hybridisierung von Nukleinsäuren

Für das CFTR-Gen sind mehr als 800 Mutationen in der Literatur beschrieben, die zum Krankheitsbild der Cystischen Fibrose führen können. Im CFTR-Gen treten drei Typen von Mutationen auf:

- Basenaustausch (hier: Punktmutationen)
- Insertionen
- Deletionen

Für alle 3 Mutationsformen sollte getestet werden, ob der jeweilige Wildtyp (pm) von der Mutation (mm) mittels Silberverstärkungsdetektion unterscheidbar ist.

Sonden und Targets wurden von der Fa. Ogham bereitgestellt.

Herstellung der Sondenarrays

Auf einer epoxidierten 3D-Objektträger (75 mm x 25 mm)-Glasoberfläche (Fa. Elipsa) wurden mit einem Micro-

Grid II-Arrayer (Fa. BioRobotics) 10 aminomodifizierte Oligonukleotide (Sonden) mit einer Länge von 16 bis 22 Nukleotiden an definierten Stellen abgelegt und kovalent immobilisiert (Arrayelemente). Die 10 Sonden gliedern sich in 5 Paare, wobei die erste jeweils den Wildtyp und die zweite die Mutation darstellt. Das Sondenpaar 1 und 2 stellt eine Punktmutation dar, das Paar 3 und 4 eine Deletion und die Paare 5/6, 7/8, 9/10 Insertionen. Die Sequenzen der Oligonukleotide waren folgende:

Sequenz in 5' - 3'-Richtung mit 3'-NH$_2$-Modifikation:

| | | |
|---|---|---|
| 1: | GATCTTCGCCTTACTG | pm |
| 2: | GATCTTCACCTTACTG | mm |
| 3: | GAAACACCAAAGATGATA | pm |
| 4: | GAAACACC GATGATA | mm |
| 5: | CTTCTAATTA TTTGGTATGT | pm |
| 6: | CTTCTAATTATTTGGTATGT | mm |
| 7: | GAGTTCTTCTAATTA TTTGG | pm |
| 8: | GAGTTCTTCTAATTATTTGG | mm |
| 9: | TTTTAGAGTTCTTCTAATTA T | pm |
| 10: | TTTTAGAGTTCTTCTAATTATT | mm |

Das Sondenpaar 3 (Wildtyp) und 4 (Deletion) beinhaltet die häufigste Mutation (70% aller Fälle), die für Cystische Fibrose kodiert.

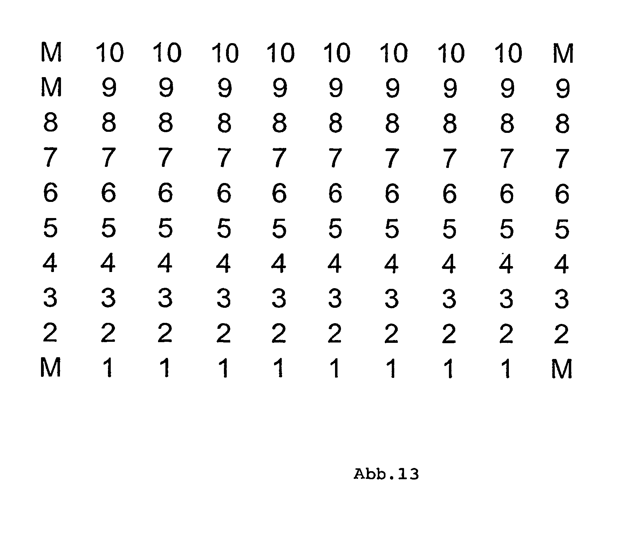

Ein einzelnes komplettes (quadratisches) Sondenarray auf der Objektträger-Oberfläche bestand aus insgesamt 10 x 10 = 100 abgelegten Sonden. Jede der 10 Oligonukleotid-Sonden wurde dabei in 8- bis 10-facher Wiederholung auf dem Sondenarray abgelegt (Array-Aufbau: siehe Abbildung 13). Die Sonden hatten einen Abstand von 0,2 mm, das gesamte Sondenarray bedeckte eine Fläche von 2 mm x 2 mm. Insgesamt konnten auf diese Weise pro Objektträger über 100 identische Sondenarrays erzeugt werden.

Das Ablegen der Sonden erfolgte aus einer jeweils 10 μM-Lösung der Oligonukleotide in 0,1 M Phosphatpuffer/5%-Natriumsulfat. Nach dem Ablegen und Eintrocknen wurden die Sonden durch 30-minütiges Backen bei 60 °C kovalent mit den Epoxid-Gruppen auf der Glasoberfläche verknüpft. Anschließend erfolgte ein Wasch- und Blockierungsprozess der Objektträger in folgender Reihenfolge:

5 min in 600 ml $H_2O$ bidest + 600 μl Triton x100

2 x 2 min in 600 ml $H_2O$ bidest + 60 μl HCL (konz.)

30 min in 100 mM KCl-Lösung
1 min in $H_2O$ bidest spülen

15 min bei 50°C in Glasschale in 75 ml $H_2O$ bidest + 25 ml Ethylenglykol +20 μl HCl (konz.) inkubieren

1 min in $H_2O$ bidest spülen
mit Druckluft trocknen

Nach Beendigung der Waschschritte und Trocknen der Objektträger wurden diese in 3,25 mm x 3,25 mm große Glasstücke (nachfolgend "Chips" genannt) zerschnitten. Auf jedem der Chips befand sich genau ein Sondenarray der Größe 2 mm x 2 mm.

_Hybridisierung und Konjuagtion der Sondenarrays_

Für die Hybridisierung der Sondenarrays standen für die 10 Oligonukleotid-Sonden 3 zu den Perfect match (pm)-Sonden komplementäre Biotin-markierte Targets zur Verfügung. Dabei deckte Target 1 das Sondenpaar 1 und 2, Target 2 das Paar 3 und 4 und Target 3 die Sondenpaare 5/6, 7/8 und 9/10 ab. Die Sequenzen der Targets lauteten:

**Target 1:** 5'-Biotin- CTCAGTAAGGCGAAGATCTT-3'

**Target 2:** 5'-Biotin- AATATCATCTTTGGTGTTTCCT-3'

**Target 3:** 5'-Biotin- GAACATACCAAATAATTAGAAGAACTCTAAAACA-3'

Die Hybridisierungs-Reaküon wurde in 6x SSPE-Puffer (52,59 g NaCl, 8,28 g $NaH_2PO_4$ x $H_2O$, 2,22 g EDTA x $2H_2O$ in 11 $H_2O$ bidest, auf pH 7,4 mit NaOH eingestellt)/0,1 % SDS in einem Gesamtvolumen von 70 μl mit allen drei Targets in einer 100 pM-Konzentration durchgeführt. Hierbei wurde ein Chip mit dem Sondenarray in die Hybridisierungslösung gegeben, 5 min bei 95 °C erhitzt, dann 60 min bei 30 °C unter Schütteln inkubiert.
Nach der 60-minütigen Hybridisierung wurde ein Streptavidin-Goldkonjugat direkt in die Hybridisierungslösung hinzugegeben und dann weitere 15 min bei 30 °C inkubiert. Als Streptavidin-Goldkonjugat wurden Goldpartikel der Größe 5 nm verwendet (British Biocell International, EM.STP5). Das Konjugat lag im Versuch in einer Konzentration von 500 pg Streptavidin/μl vor.

Nach der Hybridisierung und Konjugation wurde der Chip in ein neues Reaktionsgefäß mit 500 μl Hybridisierungspuffer (ohne Target) überführt und 10 min bei 55 °C unter Schütteln gewaschen. Anschließend wurden die Chips dann noch je 10 min in 2x SSC/0,2 % SDS (500 μl bei 30 °C), 2x SSC (500 μl bei 20 °C) und 0,2x SSC (500 μl bei 20 °C) unter Schütteln gewaschen und dann getrocknet (Eppendorf-Concentrator).

Silberverstärkung, Detektion und Auswertung

Zur Silberverstärkung wurden die Chips in einer abgeschlossenen Reaktionskammer (siehe Abbildung 1) fixiert und mit einer Silberentwicklungslösung (British Biocell International, SEKL15) überschichtet. Die Inkubationslösung wurde durch Mischen von je einem Tropfen Initiator- und Enhancer-Lösung hergestellt. Während der 30-minütigen Inkubation bei 21 °C wurde der zeitliche Verlauf der Silberverstärkung durch ein Foto pro Minute dokumentiert (als Lichtquelle diente eine rote LED).

Die Bilder wurden anschließend mit der Bildauswertungs-Software IconoClust (Fa. Clondiag) ausgewertet.

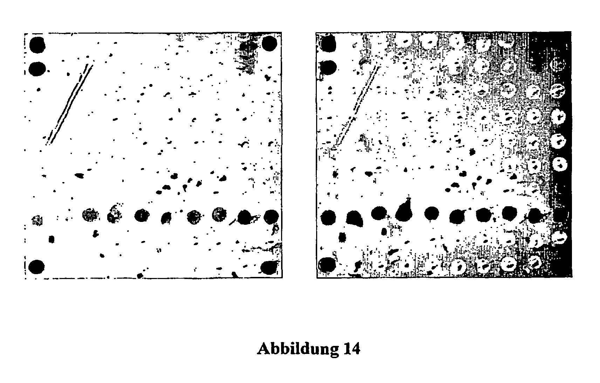

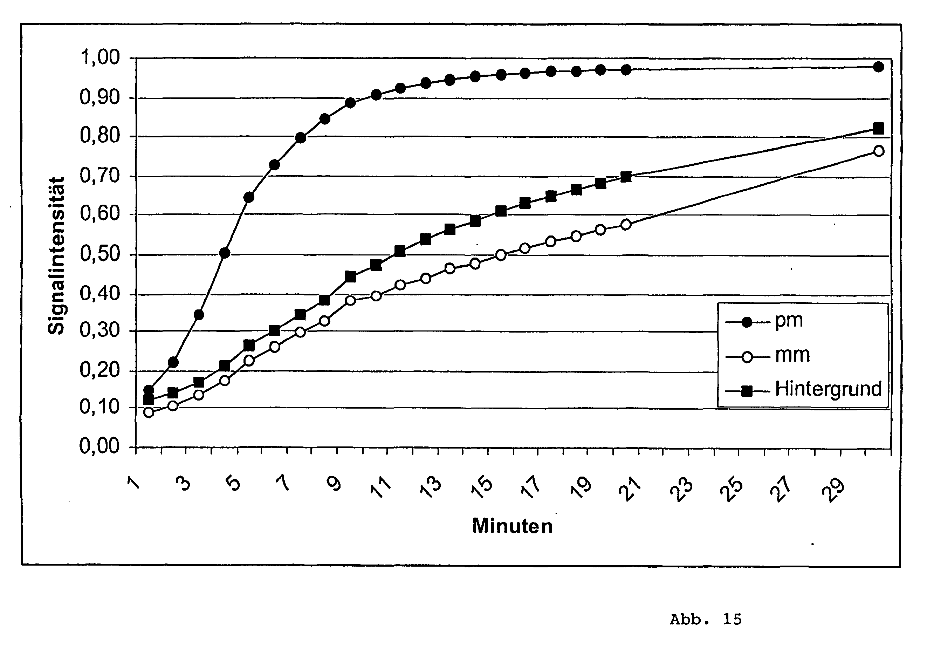

Als Beispiel sind in Abb. 14a und 14b die Chip-Fotos 5 bzw. 10 min nach Beginn der Silberverstärkung dargestellt (hybridisiert wurde mit dem Target 2). Abb. 15 zeigt den zeitlichen Verlauf dieser Reaktion.

Beispiel 7: Nachweis und Spezifität der Hybridisierung von Nukleinsäuren - Zeitreihenmessung

Für das CFTR-Gen sind mehr als 800 Mutationen in der Literatur beschrieben, die zum Krankheitsbild der Cystischen Fibrose führen können. Im CFTR-Gen treten drei Typen von Mutationen auf:

- Basenaustausch (hier: Punktmutationen)
- Insertionen
- Deletionen

Für alle 3 Mutationsformen sollte getestet werden, ob der jeweilige Wildtyp (pm) von der Mutation (mm) mittels Silberverstärkungsdetektion unterscheidbar ist. Sonden und Targets wurden von der Fa. Ogham (Münster) bereitgestellt.

Herstellung der Sondenarrays

Auf einer epoxidierten 3D-Objektträger (75 mm x 25 mm)-Glasoberfläche (Fa. Elipsa) wurden mit einem Micro-Grid II-Arrayer (Fa. BioRobotics) 10 aminomodifizierte Oligonukleotide (Sonden) mit einer Länge von 16 bis 22 Nukleotiden an definierten Stellen abgelegt und kovalent immobilisiert (Arrayelemente). Die 10 Sonden gliedern sich in 5 Paare, wobei die erste jeweils den Wildtyp und die zweite die Mutation darstellt. Das Sondenpaar 1 und 2 stellt eine Punktmutation dar, das Paar 3 und 4 eine Deletion und die Paare 5/6, 7/8, 9/10 Insertionen. Die Sequenzen der Oligonukleotide waren folgende:

Sequenz in 5' - 3'-Richtung mit 3'-NH$_2$-Modifikation:

| | | |
|---|---|---|
| 1: | GATCTTCGCCTTACTG | pm |
| 2: | GATCTTCACCTTACTG | mm |
| 3: | GAAACACCAAAGATGATA | pm |
| 4: | GAAACACC    GATGATA | mm |
| 5: | CTTCTAATTA TTTGGTATGT | pm |

6:    CTTCTAATTATTTTGGTATGT    mm

7:    GAGTTCTTCTAATTA TTTGG    pm

8:    GAGTTCTTCTAATTATTTTGG    mm

9:    TTTTAGAGTTCTTCTAATTA T    pm

10:   TTTTAGAGTTCTTCTAATTATT    mm

Das Sondenpaar 3 (Wildtyp) und 4 (Deletion) beinhaltet die häufigste Mutation (70% aller Fälle), die für Cystische Fibrose kodiert.

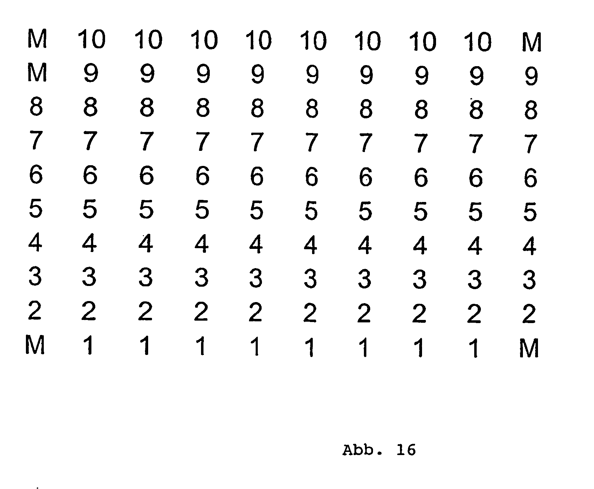

Ein einzelnes komplettes (quadratisches) Sondenarray auf der Objektträger-Oberfläche bestand aus insgesamt 10 x 10 = 100 abgelegten Sonden. Jede der 10 Oligonukleotid-Sonden wurde dabei in 8- bis 10-facher Wiederholung auf dem Sondenarray abgelegt (Array-Aufbau: siehe Abb. 16). Die Sonden hatten einen Abstand von 0,2 mm, das gesamte Sondenarray bedeckte eine Fläche von 2 mm x 2 mm. Insgesamt konnten auf diese Weise pro Objektträger über 100 identische Sondenarrays erzeugt werden.

Das Ablegen der Sonden erfolgte aus einer jeweils 10 µM-Lösung der Oligonukleotide in 0,1 M Phosphatpuffer/5%-Natriumsulfat. Nach dem Ablegen und Eintrocknen wurden die Sonden durch 30-minütiges Backen bei 60 °C kovalent mit den Epoxid-Gruppen auf der Glasoberfläche verknüpft. Anschließend erfolgte ein Wasch- und Blockierungsprozess der Objektträger in folgender Reihenfolge:

5 min in 600 ml $H_2O$ bidest + 600 µl Triton x 100

2 x 2 min in 600 ml $H_2O$ bidest + 60 µl HCL (konz.)

30 min in 100 mM KCI-Lösung
1 min in $H_2O$ bidest spülen

15 min bei 50°C in Glasschale in 75 ml $H_2O$ bidest + 25 ml Ethylenglykol +20 µl HCl (konz.) inkubieren

1 min in $H_2O$ bidest spülen
mit Druckluft trocknen

Nach Beendigung der Waschschritte und Trocknen der Objektträger wurden diese in 3,25 mm x 3,25 mm große Glasstücke (nachfolgend "Chips" genannt) zerschnitten. Auf jedem der Chips befand sich genau ein Sondenarray der Größe 2 mm x 2 mm.

Hybridisierung und Konjuagtion der Sondenarrays

Für die Hybridisierung der Sondenarrays standen für die 10 Oligonukleotid-Sonden 3 zu den Perfect match (pm)-Sonden komplementäre Biotin-markierte Targets zur Verfügung. Dabei deckte Target 1 das Sondenpaar 1 und 2, Target 2 das Paar 3 und 4 und Target 3 die Sondenpaare 5/6, 7/8 und 9/10 ab. Die Sequenzen der Targets lauteten:

Target 1: 5'-Biotin- CTCAGTAAGGCGAAGATCTT-3'

Target 2: 5'-Biotin- AATATCATCTTTGGTGTTTCCT-3'

Target 3: 5'-Biotin- GAACATACCAAATAATTAGAAGAACTCTAAAACA-3'

Die Hybridisierungs-Reaktion wurde in 6x SSPE-Puffer (52,59 g NaCl, 8,28 g $NaH_2PO_4$ x $H_2O$, 2,22 g EDTA x $2H_2O$ in 1l $H_2O$ bidest, auf pH 7,4 mit NaOH eingestellt)/0,1 % SDS in einem Gesamtvolumen von 70 µl mit dem Target 3 in einer 100 nM- und 1 nM-Konzentration durchgeführt. Hierbei wurde ein Chip mit dem Sondenarray in die Hybridisierungslösung gegeben, 5 min bei 95 °C erhitzt, dann 60 min bei 55°C unter Schütteln inkubiert.

Anschließend wurden die hybridisierten Chips je 10 min in 2 x SSC/0,2 % SDS (500 µl bei 30°C), 2 x SSC (500 µl bei 20°C) und 0,2 x SSC (500 µl bei 20°C) unter Schütteln gewaschen. In einem neuen Reaktionsgefäß schloss sich eine 15 minütige Inkubation (bei 30°C) mit einem Streptavidin-Goldkonjugat an. Als Streptavidin-Goldkonjugat wurden Goldpartikel der Größe 5 nm verwendet (British Biocell International, EM.STP5). Das Konjugat lag im Versuch in einer Konzentration von 125 pg Streptavidin/µl vor.

Nach der Konjugation wurde der Chip in ein neues Reaktionsgefäß überführt und je 10 min in 2 x SSC/0,2 % SDS (500 µl bei 30°C), 2 x SSC (500 µl bei 20°C) und 0,2 x SSC (500 µl bei 20°C) unter Schütteln gewaschen und dann getrocknet (Eppendorf-Concentrator).

Silberverstärkung, Detektion und Auswertung

Zur Silberverstärkung wurden die Chips in einer abgeschlossenen Reaktionskammer (siehe hierzu Abbildung 1) fixiert und mit einer Silberentwicklungslösung (British Biocell International, SEKL15) überschichtet. Die Inkubationslösung wurde durch Mischen von je einem Tropfen Initiator- und Enhancer-Lösung hergestellt. Während der 20-minütigen Inkubation bei 27 °C wurde der zeitliche Verlauf der Silberverstärkung durch ein Fotoserie (alle 10 s ein Bild) dokumentiert (als Lichtquelle diente eine rote LED). Die Bilder wurden anschließend mit der Bildauswertungs-Software IconoClust (Fa. Clondiag) ausgewertet.

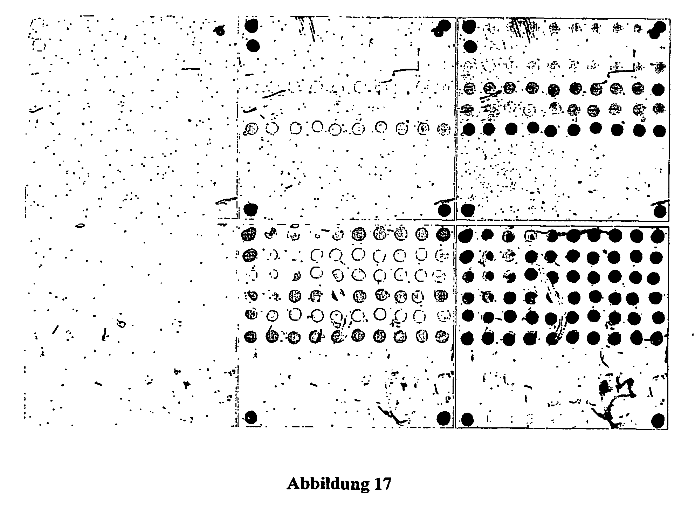

Die Ergebnisse sind in den Abbildungen 17 bis 20 sowie Tabelle 2 dargestellt. Aus dem Vergleich der für die jeweilige Sonde bei einem Target bestimmter Konzentration typischen linearen Regressionsgeraden, die im Bereich des exponentiellen Anstiegs der Kurve ermittelt werden, läßt sich eine unbekannte Target-Konzentration abschätzen. Vorraussetzung hierfür sind gleiche Mengen eingesetzten Konjugates, gleiche Konzentration der immobilisierten Sonden sowie gleiche Versuchstemperatur.

Tabelle 2:

| Lineare Regressionsgleichungen für ausgewählte Sonden (Array-Elemente) sowie den Chip-Hintergrund. Der Anstieg der jeweiligen Regressionsgeraden ist fett gedruckt (x: Zeit in Minuten nach Beginn der Silberverstärkung, y: Signalintensität im gültigen Minutenbereich, Hybridisierung mit Target 3 in den Konzentrationen 100 nM und 1 nM). | | | | | |
|---|---|---|---|---|---|
| *Sondenarray-Element* | *Target Konzentration* | *Bereich Minuten* | *Gleichung f(x)* | *$R^2$* | *Standard- Fehler* |
| **Hintergrund** | 100 nM | 1-20 | y= 0,0551+ **(0,013 * x)** | 0,971 | 0,014 |
| **Hintergrund** | 1 nM | 1-20 | y= 0,0161+ **(0,013 * x)** | 0,984 | 0,01 |
| **Sonde 5 pm** | 100 nM | 4-13 | y= -0,263+ **(0,0740 * x)** | 0,993 | 0,021 |
| **Sonde 5 pm** | 1 nM | 4-13 | y= -0,259+ **(0,0677 * x)** | 0,989 | 0,023 |

Tabelle 2: (fortgesetzt)

| Lineare Regressionsgleichungen für ausgewählte Sonden (Array-Elemente) sowie den Chip-Hintergrund. Der Anstieg der jeweiligen Regressionsgeraden ist fett gedruckt (x: Zeit in Minuten nach Beginn der Silberverstärkung, y: Signalintensität im gültigen Minutenbereich, Hybridisierung mit Target 3 in den Konzentrationen 100 nM und 1 nM). | | | | | |
|---|---|---|---|---|---|
| *Sondenarray-Element* | *Target Konzentration* | *Bereich Minuten* | *Gleichung f(x)* | *$R^2$* | *Standard- Fehler* |
| **Sonde 6 mm** | 100 nM | 4-13 | y= -0,246+ **(0,0647 * x)** | 0,991 | 0,02 |
| **Sonde 6 mm** | 1 nM | 4-13 | y=-0,153+ **(0,0417 * x)** | 0,969 | 0,024 |

Abbildungen

Abbildung 1: Vorrichtung zum qualitativen und/oder quantitativen Nachweis von Wechselwirkungen zwischen Sonden und Targets.

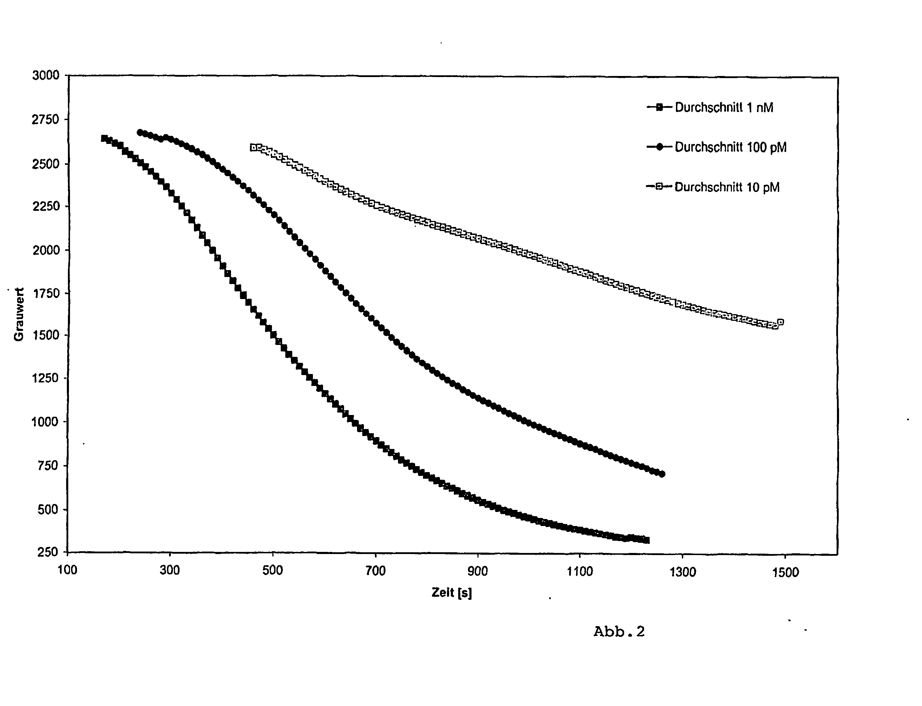

Abbildung 2: Wiedergabe des zeitlichen Verlaufs der in Abbildung 3 dargestellten Hybridisierungsergebnisse.

Abbildung 3: Darstellung der Ergebnisse der Hybridisierung.

A - Hybridisierung des in einer Konzentration von 10 nM vorliegenden Targets,

B - Hybridisierung des in einer Konzentration von 1 nM vorliegenden Targets,

C - Hybridisierung des in einer Konzentration von 100 pM vorliegenden Targets und

D - Hybridisierung des in einer Konzentration von 10 pM vorliegenden Targets.

Abbildung 4: Nachweis der Hybridisierung von genomischer RNA aus *Corynebacterium glutamicum* gegen ein Sondenarray von 356 Sonden - resultierendes Muster nach 15minütiger Inkubation.

Abbildung 5: Aufbau eines Arrays der Größe 2 mm x 2 mm in einem 10 x 10 = 100 Sondenarray.

Die Zahlen 1-16 stehen jeweils für eine Oligonukleotid-Sonde, die in 5- bzw. 6-facher Wiederholung auf dem array abgelegt ist; "M" steht für ein Markengemisch (enthält u.a. ein immobilisiertes Biotin-markiertes Oligonukleotid); 1 Position auf + dem Array ist nicht belegt.

Abbildung 6: Sondenarray nach erfolgter Hybridisierung, Konjugation und Silberverstärkung (zum Arrayaufbau vgl. Abb. 5).

a) linkes Bild: Target 9b (100 nM)-Hybridisierung bei 30 °C; 1. Waschschritt bei 60 °C; Streptavidin-Goldkonjugat (500 pg/μl); Silberverstärkung: 10 min bei 25 °C

Außer der spezifischen Sonde 9 (und den Marken) ist noch ein schwach unspezifisches Signal der Sonde 13 erkennbar.

b) rechtes Bild: Target 9b (100 pM)-Hybridisierung bei 30 °C mit anschließender direkter Streptavidin-Goldkonjugat Zugabe (500 pg/μl); 1. Waschschritt bei 60 °C, Silberverstärkung: 10 min bei 25 °C.

Abbildung 7: Aufbau eines Arrays der Größe 2 mm x 2 mm in einem 10 x 10 = 100 Sondenarray.

Die Zahlen 1-10 stehen jeweils für eine Oligonukleotid-Sonde, die in 8- bis 10-facher Wiederholung auf dem Array abgelegt ist; "M" steht für ein Markengemisch (enthält u.a. ein immobilisiertes Biotin-markiertes Oligonukleotid).

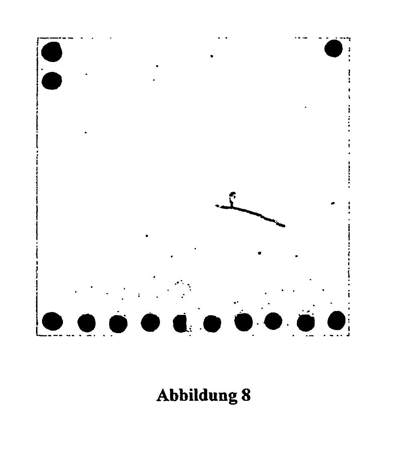

Abbildung 8: Sondenarray nach erfolgter Hybridisierung, Konjugation und Silberverstärkung (zum Arrayaufbau vgl. Abb. 7).

Target 1 (100 pM)-Hybridisierung bei 30 °C mit anschließender direkter Streptavidin-Goldkonjugat Zugabe (500 pg/μl); 1. Waschschritt bei 55 °C, Silberverstärkung: 10 min bei 25 °C.

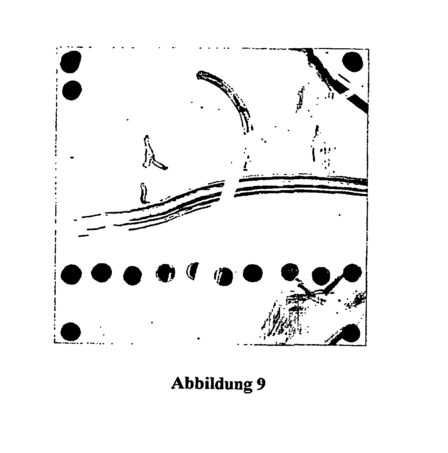

Abbildung 9: Sondenarray nach erfolgter Hybridisierung, Konjugation und Silberverstärkung (zum Arrayaufbau vgl. Abb. 1).

Target 2 (100 pM)-Hybridisierung bei 30 °C mit anschließender direkter Streptavidin-Goldkonjugat Zugabe (500 pg/μl); 1. Waschschritt bei 55 °C, Silberverstärkung: 10 min bei 25 °C.

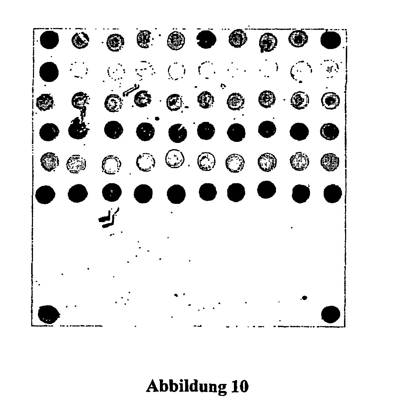

Abbildung 10: Sondenarray nach erfolgter Hybridisierung, Konjugation und Silberverstärkung (zum Arrayaufbau vgl. Abb. 1).

Target 3 (100 pM)-Hybridisierung bei 30 °C mit anschließender direkter Streptavidin-Goldkonjugat Zugabe (500 pg/μl); 1. Waschschritt bei 55°C, Silberverstärkung: 10 min bei 25°C.

Abbildung 11: Aufbau eines Arrays der Größe 2 mm x 2 mm in einem 10 x 10 = 100 Sondenarray.

Die Zahlen 1-16 stehen jeweils für eine Oligonukleotid-Sonde, die in 5- bzw. 6-facher Wiederholung auf dem Array abgelegt ist; "M" steht für ein Markengemisch (enthält u.a. ein immobilisiertes Biotin-markiertes Oligonukleotid); 1 Position auf dem Array ist nicht belegt.

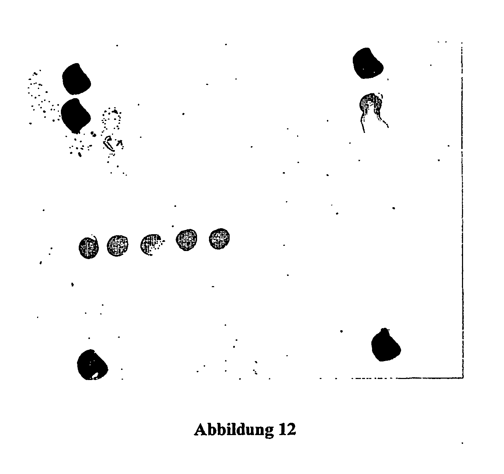

Abbildung 12: Sondenarray nach erfolgter Hybridisierung, Konjugation und Silberverstärkung von Beispiel 5 (zum Arrayaufbau vgl. Abb. 11).

Target 9c (1 nM)- 60 min Hybridisierung bei 30 °C; dann Zugabe von T20-Nanogold (1:100 Verdünnung) und weitere Inkubation für 30 min bei 30 °C, 1.Waschschritt bei 55 °C; Silberverstärkung: 10 min bei 25 °C.

Außer dem starken spezifischen Signal der Sonde 9 (und den Marken) liefern auch die übrigen Sonden ein schwaches Signal; die teilweise verlaufenenen und inhomogenen Spots kamen durch beim Ablegen der Sonden durch Verunreinigung der Nadel des Arrayers zustande.

Abbildung 13: Aufbau eines Arrays der Größe 2 mm x 2 mm in einem 10 x 10 =100 Sondenarray

Die Zahlen 1-10 stehen jeweils für eine Oligonukleotid-Sonde, die in 8- bis 10-facher Wiederholung auf dem Array abgelegt ist; "M" steht für ein Markengemisch (enthält u.a. ein immobilisiertes Biotin-markiertes Oligonukleotid).

Abbildung 14: Sondenarray nach erfolgter Hybridisierung und Konjugation (zum Arrayaufbau vgl. Abb. 1).

Target 2 (100 pM)-Hybridisierung bei 30 °C mit anschließender direkter Streptavidin-Goldkonjugat Zugabe (500 pg/μl); 1. Waschschritt bei 55 °C

    a) linkes Bild: 5 min nach Beginn Silberverstärkung

    b) rechtes Bild: 10 min nach Beginn Silberverstärkung

Abbildung 15: Zeitlicher Verlauf der Silberverstärkung (vgl. hierzu Abbildungen 13 und 14).

Minütliche Messung, jeder Meßpunkt ist ein Mittelwert aus 10 Spot-Wiederholungen

pm: Perfect match-Sonde (Sonden-Nr. 3)

mm: Mismatch-Sonde (Sonden-Nr. 4)

Target 2 (100 pM)-Hybridisierung bei 30 °C mit anschließender direkter Streptavidin-Goldkonjugat Zugabe (500 pg/μl)

Abbildung 16: Aufbau eines Arrays der Größe 2 mm x 2 mm in einem 10 x 10 = 100 Sondenarray

Die Zahlen 1-10 stehen jeweils für eine Oligonukleotid-Sonde, die in 8- bis 10-facher Wiederholung auf dem array abgelegt ist; "M" steht für ein Markengemisch (enthält u.a. ein immobilisiertes Biotin-markiertes Oligonukleotid in der Konzentration 10 μM).

Das hybridisierte Target 3 war komplementär zu den Sonden 5, 7, 9 (jeweils perfect match) und den Sonden 6, 8, 10 (jeweils mismatch mit einer Insertion).

Abbildung 17: Sondenarray nach erfolgter Hybridisierung und Konjugation (zum Arrayaufbau vgl. Abb. 16)

Die Bilder sind von links nach rechts jeweils 5 min, 10 min und 20 min nach Beginn der Silberverstärkung aufgenommen

oben: Target 3 (1 nM)-Hybridisierung

unten: Target 3 (100 nM)-Hybridisierung

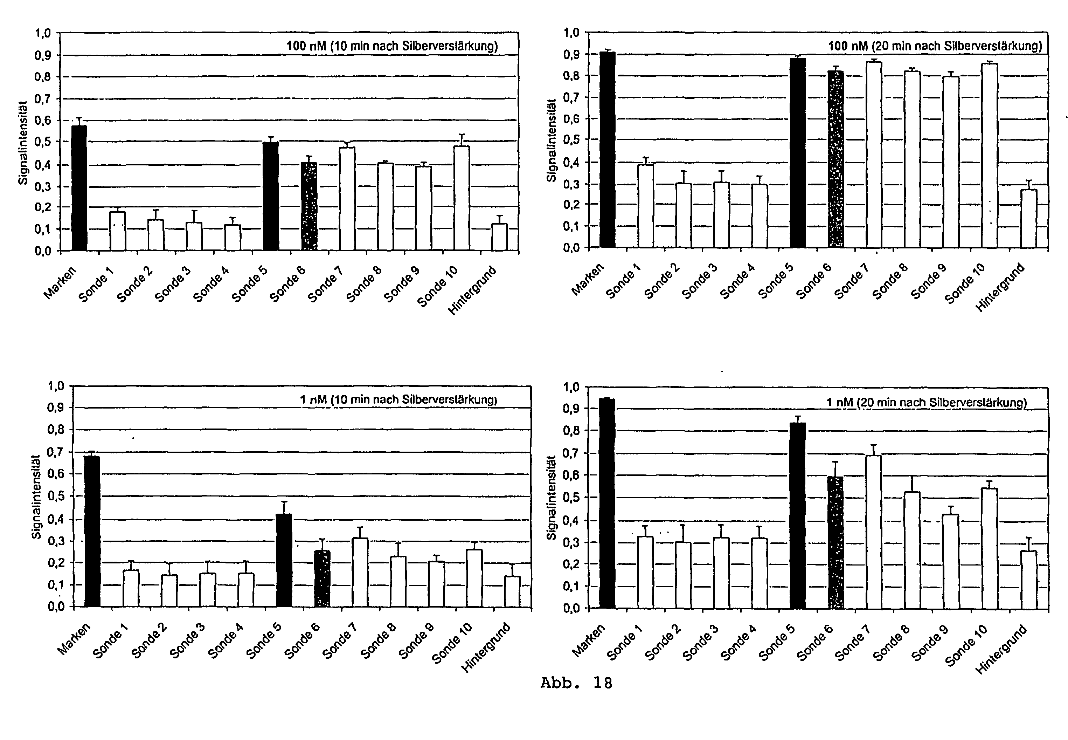

Abbildung 18: Signalintensitäten nach unterschiedlich langer Silberverstärkung bei 2 verschiedenen Targetkonzentrationen (vgl. mit Bildern aus Abbildung 17)

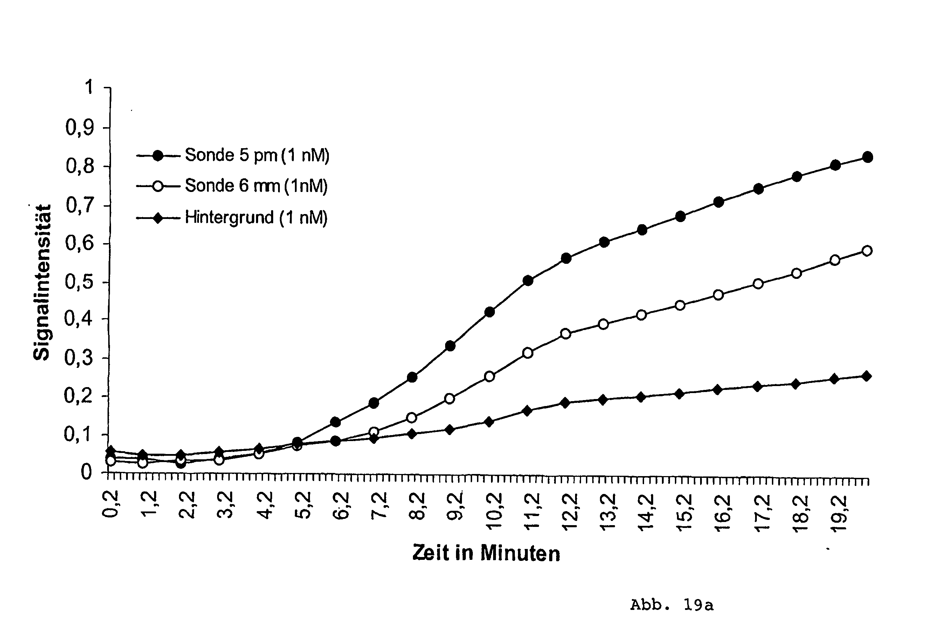

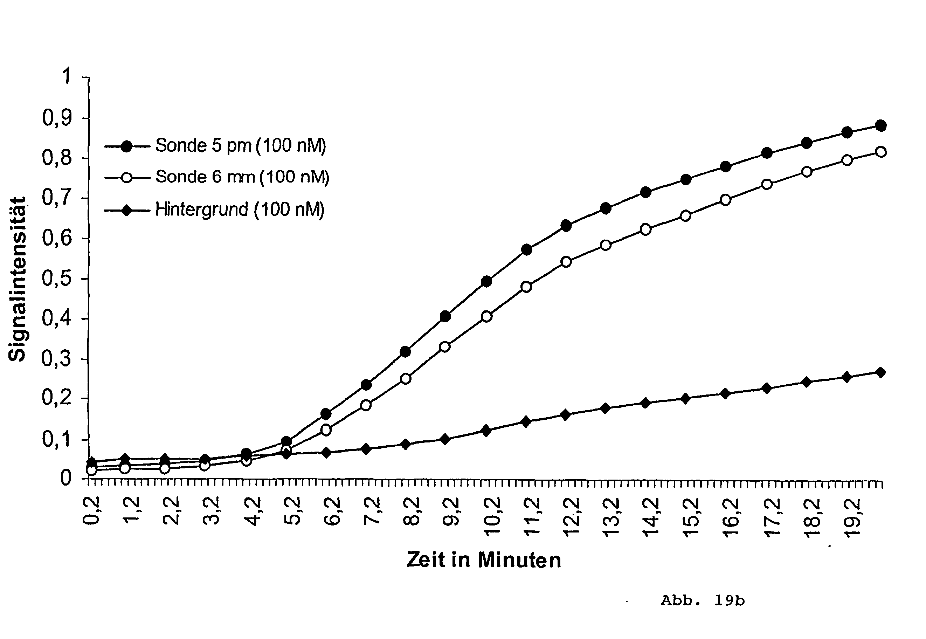

Abbildung 19 a (oben) und b(unten): Zeitlicher Verlauf der Silberverstärkung am Beispiel der Sonden 5 und 6 (vgl. hierzu Abb. 16 bis 18)

Jeder Sonden-Meßpunkt ist ein Mittelwert aus 7 bis 10 Spot-Wiederholungen.

pm: Perfect match-Sonde (Sonden-Nr. 5)

mm: Mismatch-Sonde (Sonden-Nr. 6)

a: Target 3 (1 nM): Hybridisierung bei 55 °C ; Streptavidin-Goldkonjugat (125 pg/μl)

b: Target 3 (100 nM): Hybridisierung bei 55 °C ; Streptavidin-Goldkonjugat (125 pg/μl)

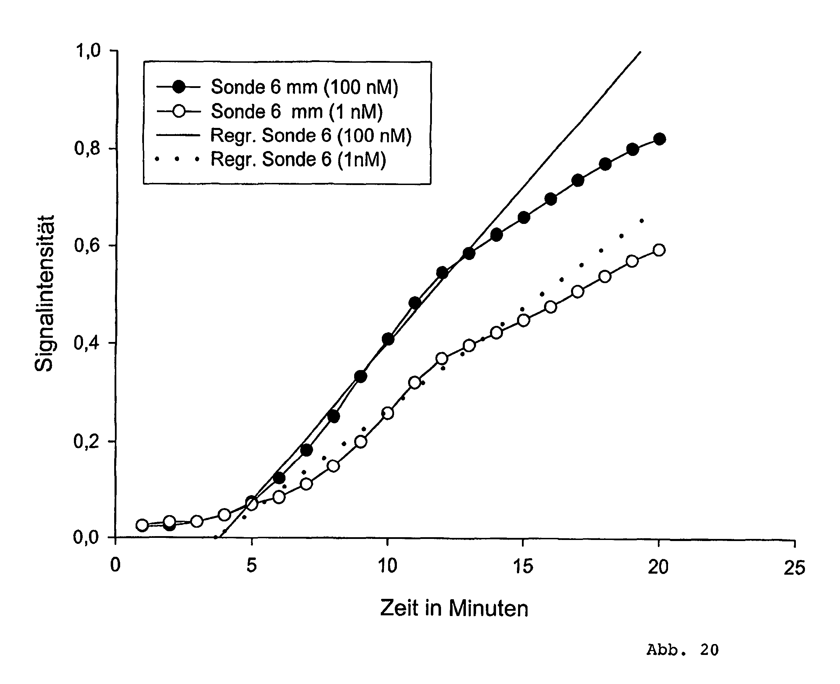

Abbildung 20: Ermittelte lineare Regressionsgeraden nach Hybridisierung mit Target 3 (100 nM und 1 nM) für zwei ausgewählte Sonden des Sondenarrays, gültig für den Bereich von 4 bis 13 Minuten nach Beginn der Silberverstärkung.

**Patentansprüche**

**1.** Verfahren zum qualitativen und/oder quantitativen Nachweis von Targets aus einer Probe durch molekulare Wechselwirkungen zwischen Sonden und Targets auf Sonden-Arrays, umfassend die folgenden Schritte:

    a) Herstellung eines Sondenarrays mit an definierten Stellen immobilisierten Sonden;

    b) Wechselwirkung der Targets mit den auf dem Sondenarray angeordneten Sonden;

c) Durchführung einer Reaktion, die zu einem Niederschlag an Array-Elementen führt, an denen eine Wechselwirkung erfolgt ist;

d) Detektion des zeitlichen Verlaufs der Niederschlagsbildung an den Array-Elementen in Form von Signalintensitäten;

e) Bestimmung einer virtuellen Signalintensität für ein Array-Element anhand einer Kurvenfunktion, die die Niederschlagsbildung als Funktion der Zeit beschreibt.

2. Verfahren nach Anspruch 1,
**dadurch gekennzeichnet, daß** die virtuelle Signalintensität für ein Array-Element in Abhängigkeit von der Steigung einer Regressionsgerade, die die Niederschlagsbildung als Funktion der Zeit beschreibt, bestimmt wird.

3. Verfahren nach Anspruch 2,
**dadurch gekennzeichnet, daß** die Regressionsgerade im Bereich des exponentiellen Anstiegs der Niederschlagsbildung mit der Zeit auf dem Array-Element ermittelt wird.

4. Verfahren nach Anspruch 2 oder 3,
**dadurch gekennzeichnet, daß** die virtuelle Signalintensität für ein Array-Element durch Multiplikation der detektierten Signalintensität zu einem bestimmten Zeitpunkt, vorzugsweise der Signalintensität der letzten Messung, mit der Steigung der für das Array-Element ermittelten Regressionsgerade sowie mit der Meßdauer zu dem bestimmten Zeitpunkt bestimmt wird.

5. Verfahren nach einem der vorangehenden Ansprüche,
**dadurch gekennzeichnet, daß** in der Probe ein Referenz-Target mit bekannter Konzentration vorliegt, das mit mindestens einer Sonde des Sondenarrays wechselwirkt.

6. Verfahren nach einem der vorangehenden Ansprüche,
**dadurch gekennzeichnet, daß** zur Detektion des zeitlichen Verlaufs der Niederschlagsbildung an den Array-Elementen die Signalintensitäten mindestens jede Minute, vorzugsweise alle 30 Sekunden, besonders bevorzugt alle 10 Sekunden aufgenommen werden.

7. Verfahren nach einem der vorangehenden Ansprüche,
**dadurch gekennzeichnet, daß** die Reaktion, die zur Bildung eines Niederschlags an den Array-Elementen führt, die Umwandlung eines löslichen Substrats in ein unlösliches Produkt in Gegenwart eines mit den Targets gekoppelten Katalysators ist.

8. Verfahren nach Anspruch 7,
**dadurch gekennzeichnet, daß** der Katalysator ein Enzym ist.

9. Verfahren nach Anspruch 7 oder 8,
**dadurch gekennzeichnet, daß** das Enzym ausgewählt wird aus der Gruppe, bestehend aus Meerettichperoxidase, alkalischer Phosphatase und Glucoseoxidase.

10. Verfahren nach einem der Ansprüche 7 bis 9,
**dadurch gekennzeichnet, daß** das lösliche Substrat ausgewählt wird aus der Gruppe, bestehend aus 3,3'-Diaminobenzidin, 4-Chlor-1-naphthol, 3-Amino-9-ethylcarbazol, p-Phenylendiamin-HCl/Pyrocatechol, 3,3',5,5'-Tetramethylbenzidin, Naphthol/Pyronin, Bromchlorindoylphosphat, Nitrotetraazoliumblau und Phenazinmethosulfat.

11. Verfahren nach einem der Ansprüche 1 bis 6,
**dadurch gekennzeichnet, daß** die Reaktion, die zur Bildung eines Niederschlags an den Array-Elementen führt, die Umwandlung eines löslichen Substrats in einen metallischen Niederschlag ist.

12. Verfahren nach Anspruch 11,
**dadurch gekennzeichnet, daß** die zur Bildung eines Niederschlags an den Array-Elementen führende Reaktion die chemische Reduktion einer Silberverbindung, vorzugsweise Silbernitrat, Silberlactat, Silberacetat oder Silbertartrat, zu elementarem Silber ist.

13. Verfahren nach Anspruch 12,
**dadurch gekennzeichnet, daß** das Reduktionsmittel ausgewählt wird aus der Gruppe bestehend aus Formaldehyd und Hydrochinon.

**14.** Verfahren nach einem der Ansprüche 11 bis 13,
**dadurch gekennzeichnet, daß** die Umwandlung eines löslichen Substrats in einen metallischen Niederschlag in Gegenwart von mit den Targets gekoppelten Metallclustem bzw. kolloidalen Metallpartikeln erfolgt.

**15.** Verfahren nach Anspruch 14,
**dadurch gekennzeichnet, daß** die Umwandlung eines löslichen Substrats in einen metallischen Niederschlag in Gegenwart von Goldclustem oder kolloidalen Goldpartikeln erfolgt.

**16.** Verfahren nach einem der Ansprüche 11 bis 13,
**dadurch gekennzeichnet, daß** die die Umwandlung eines löslichen Substrats in einen metallischen Niederschlag in Gegenwart von mit den Targets gekoppelten Polyanionen erfolgt.

**17.** Verfahren nach einem der Ansprüche 7 bis 16,
**dadurch gekennzeichnet, daß** die Katalysatoren bzw. Metallcluster bzw. kolloidalen Metallpartikel bzw. Polyanionen an die Targets vor, während oder nach der Wechselwirkung mit den Sonden gekoppelt werden.

**18.** Verfahren nach einem der Ansprüche 7 bis 17,
**dadurch gekennzeichnet, daß** die Kopplung der Enzyme bzw. Metallcluster bzw. kolloidalen Metallpartikel bzw. Polyanionen an die Targets direkt oder über an die Targets gekoppelte Ankermoleküle erfolgt.

**19.** Verfahren nach Anspruch 18,
**dadurch gekennzeichnet, daß** das Ankermolekül ausgewählt wird aus der Gruppe bestehend aus Streptavidin oder einem Antikörper.

**20.** Verfahren nach einem der Ansprüche 1 bis 6,
**dadurch gekennzeichnet, daß** die Reaktion, die zur Bildung eines Niederschlags an den Array-Elementen führt, die Bindung eines spezifischen Bindungspartners an ein an das Target gekoppeltes Ankermolekül darstellt.

**21.** Verfahren nach Anspruch 20,
**dadurch gekennzeichnet, daß** die Bindungspartner/Ankermolekül-Paare ausgewählt werden aus der Gruppe, bestehend aus Biotin/Avidin bzw. Streptavidin bzw. Anti-Biotin-Antikörper, Digoxigenin/Antidigoxigenin-Immunoglobulin, FITC/Anti-FITC-Immunoglobulin und DNP/Anti-DNP-immunoglobulin.

**22.** Verfahren nach einem der vorangehenden Ansprüche,
**dadurch gekennzeichnet, daß** die Targets direkt mit einer Markierung versehen werden.

**23.** Verfahren nach einem der Ansprüche 1 bis 21,
**dadurch gekennzeichnet, daß** eine Markierung der Targets über Sandwich-Reaktionen bzw. Sandwich-Hybridisierungen mit der mit den Targets wechselwirkenden Sonden und einer markierten Verbindung erfolgt.

**24.** Verfahren nach einem der Ansprüche 1 bis 21,
**dadurch gekennzeichnet, daß** eine Markierung der Targets durch Anfügen einer homopolymeren Nukleotid-Sequenz an die Targets unter Bildung einer fortlaufenden Sequenz und anschließende Sandwich-Hybridisierung mit einem zur homopolymeren Nukleotid-Sequenz komplementären markierten Oligonukleotid erfolgt.

**25.** Verfahren nach einem der vorangehenden Ansprüche,
**dadurch gekennzeichnet, daß** die Wechselwirkung zwischen dem Target und der Sonde eine Hybridisierung zwischen zwei Nukleotidsequenzen ist.

**26.** Verfahren nach einem der Ansprüche 1 bis 24,
**dadurch gekennzeichnet, daß** die Wechselwirkung zwischen dem Target und der Sonde eine Reaktion zwischen einer Antigenstruktur und dem entsprechenden Antikörper oder einem hypervariablen Abschnitt davon ist.

**27.** Verfahren nach einem der vorangehenden Ansprüche,
**dadurch gekennzeichnet, daß** die Wechselwirkung zwischen dem Target und der Sonde eine Reaktion zwischen einem Rezeptor und einem entsprechenden Liganden ist.

**28.** Verfahren nach einem der vorangehenden Ansprüche,
**dadurch gekennzeichnet, daß** der Nachweis der Gegenwart eines Niederschlags auf einem Array-Element durch Reflexion, Absorption oder Diffusion eines Lichtstrahls, vorzugsweise eines Laserstrahls oder einer Leuchtdiode, durch den Niederschlag erfolgt.

**29.** Verfahren nach einem der Ansprüche 1 bis 27,
**dadurch gekennzeichnet, daß** der Nachweis der Gegenwart eines Niederschlags auf einem Array-Element elektrisch erfolgt.

**30.** Verfahren nach Anspruch 29,
**dadurch gekennzeichnet, daß** der elektrische Nachweis über Leitfähigkeitsmessungen, Kapazitätsmessungen oder Potentialmessungen erfolgt.

**31.** Verfahren nach einem der Ansprüche 1 bis 27,
**dadurch gekennzeichnet, daß** der Nachweis der Gegenwart eines Niederschlags auf einem Array-Element durch Autoradiographie, Fluorographie und/oder indirekte Autoradiographie erfolgt.

**32.** Verfahren nach einem der Ansprüche 1 bis 27,
**dadurch gekennzeichnet, daß** der Nachweis der Gegenwart eines Niederschlags auf einem Array-Element durch Rasterelektronenmikroskopie, Elektronensonden-Mikroanalyse (EPMA), magnetooptische Kerr-Mikroskopie, magnetic force-Mikroskopie (MFM), atomic force-Mikroskopie (AFM), Messung des Mirage-Effekts, Rastertunnelmikroskopie (STM) und/oder Ultraschall-Reflexions-Tomographie erfolgt.

**33.** Verfahren nach einem der vorangehenden Ansprüche, umfassend die folgenden Schritte:

- Detektion des zeitlichen Verlaufs der Niederschlagsbildung an den Array-Elementen durch Aufnahme von Bildern durch eine Kamera;
- Umwandlung der in den Bildern enthaltenen analogen Information in digitale Form;
- Berechnung einer virtuellen Signalintensität für jedes Array-Element anhand einer Kurvenfunktion, die die Niederschlagsbildung als Funktion der Zeit beschreibt;
- Umwandlung der virtuellen Signalintensitäten in ein künstliches Bild, das die virtuellen Signalintensitäten aller Array-Elemente darstellt.

**34.** Vorrichtung zur Durchführung des Verfahrens nach einem der Ansprüche 1 bis 33, umfassend:

a) ein Array-Substrat mit Sonden-Array,
b) eine Reaktionskammer,
c) eine Vorrichtung zur Detektion eines Niederschlags auf einem Array-Element, an dem eine Wechselwirkung zwischen Targets und Sonden stattgefunden hat, und
d) einen Computer, der programmiert ist, um:

- die von der Detektionsvorrichtung aufgenommenen Signalintensitäten zu sammeln;
- die Verarbeitung der sukzessive aufgenommenen Signalintensitäten derart zu gewährleisten, daß der zeitliche Verlauf der Niederschlagsbildung mit der Zeit auf einem Array-Element bestimmt wird und eine virtuelle Signalintensität anhand einer Kurvenfunktion bestimmt wird, die die Niederschlagsbildung als Funktion der Zeit beschreibt; und
- ggf. die Umwandlung der virtuellen Signalintensitäten in ein analoges Bild zu gewährleisten.

**35.** Vorrichtung nach Anspruch 34,
**dadurch gekennzeichnet, daß** die Detektionsvorrichtung eine Kamera ist.

**36.** Vorrichtung nach Anspruch 35,
**dadurch gekennzeichnet, daß** die Kamera eine CCD- oder CMOS-Kamera ist.

**37.** Vorrichtung nach einem der Ansprüche 34 bis 36,
**dadurch gekennzeichnet, daß** die Vorrichtung zusätzlich eine Lichtquelle umfaßt.

**38.** Vorrichtung nach Anspruch 37,

**dadurch gekennzeichnet, daß** die Lichtquelle ausgewählt wird aus der Gruppe, bestehend aus einem Laser, einer Licht-emittierenden Diode (LED) und einer Hochdrucklampe.

39. Vorrichtung nach einem der Ansprüche 24 bis 38,
**dadurch gekennzeichnet, daß** die Vorrichtung als hochintegrierte autonome Einheit vorliegt.

**Claims**

1. A method for the qualitative and/or quantitative detection of targets from a sample by molecular interactions between probes and targets on probe arrays, comprising the following steps:

   a) preparation of a probe array, with probes immobilised at defined sites;
   b) interaction of the targets with the probes arranged on the probe array;
   c) performance of a reaction which leads to a precipitate on array elements on which an interaction has occurred;
   d) detection of the time course of the formation of the precipitate on the array elements in the form of signal intensities;
   e) determination of a virtual signal intensity for an array element on the basis of a curve function which describes the formation of the precipitate as a function of time.

2. The method according to claim 1,
**characterised by** the virtual signal intensity for an array element being determined in dependency on the gradient of a regression line which describes the formation of the precipitate as a function of time.

3. The method according to claim 2,
**characterised by** the regression line being determined in the phase of the exponential increase in formation of the precipitation with time on the array element.

4. The method according to Claims 2 or 3,
**characterised by** the virtual signal intensity for an array element being determined by multiplication of the detected signal intensity at a defined time point, preferably of the signal intensity of the last measurement, with the gradient of the regression line which has been determined for the array element and with the duration of measurement at the defined time point.

5. The method according to any of the preceding claims,
**characterised by** a reference target being present in the sample at a known concentration which interacts with at least one probe in the probe array.

6. The method according to any of the preceding claims,
**characterised by** the signal intensities for detection of the time course of the formation of precipitate on the array elements being recorded at least each minute, preferably every 30 seconds, more preferably every 10 seconds.

7. The method according to any of the preceding claims,
**characterised by** the reaction which leads to the formation of a precipitate on the array elements being the conversion of a soluble substrate into an insoluble product in the presence of a catalyst which is coupled to the targets.

8. The method according to Claim 7,
**characterised by** the catalyst being an enzyme.

9. The method according to Claims 7 or 8,
**characterised by** the enzyme being selected from the group consisting of horseradish peroxidase, alkaline phosphatase and glucose oxidase.

10. The method according to any of the Claims 7 to 9,
**characterised by** the soluble substrate being selected from the group consisting of 3,3'-diaminobenzidine, 4-chlor-1-naphthol, 3-amino-9-ethylcarbazole, p-phenylenediamine-HCl/pyrocatechol, 3,3',5,5'-tetramethylbenzidine, naphthol/pyronine, bromchlorindoylphosphate, nitrotetraazolium blue and phenazine methosulphate.

**11.** The method according to any of the Claims 1 to 6,
**characterised by** the reaction which leads to the formation of a precipitate on the array elements being the conversion of a soluble substrate into a metallic precipitate.

**12.** The method according to Claim 11,
**characterised by** the reaction which leads to the formation of a precipitate on the array elements being the chemical reduction of a silver compound, preferably silver nitrate, silver lactate, silver acetate or silver tartrate, to elemental silver.

**13.** The method according to Claim 12,
**characterised by** the reducing agent being selected from the group consisting of formaldehyde and hydroquinone.

**14.** The method according to any of the Claims 11 to 13,
**characterised by** the conversion of a soluble substrate into a metallic precipitate taking place in the presence of metal clusters or colloidal metal particles which are coupled to the targets.

**15.** The method according to Claim 14,
**characterised by** the conversion of a soluble substrate into a metallic precipitate taking place in the presence of gold clusters or colloidal gold particles.

**16.** The method according to any of the Claims 11 to 13,
**characterised by** the conversion of a soluble substrate into a metallic precipitate taking place in the presence of polyanions which are coupled to the targets.

**17.** The method according to any of the Claims 7 to 16,
**characterised by** the catalysts or metal clusters or colloidal metal particles or polyanions being coupled to the targets, before, during or after the interaction with the probes.

**18.** The method according to any of the Claims 7 to 17,
**characterised by** the coupling of the enzymes or metal clusters or colloidal metal particles or polyanions to the targets being carried out directly or through anchor molecules which are coupled to the targets.

**19.** The method according to Claim 18,
**characterised by** the anchor molecule being selected from the group consisting of streptavidin or an antibody.

**20.** The method according to any of the Claims 1 to 6,
**characterised by** the reaction which leads to the formation of a precipitate on the array elements being the binding of a specific binding partner to an anchor molecule which is coupled to the target.

**21.** The method according to Claim 20,
**characterised by** the binding partner/anchor molecule pairs being selected from the group consisting of biotin/ avidin or streptavidin or anti-biotin antibody, digoxigenin/anti-digoxigenin immunoglobulin, FITC/anti-FITC immunoglobulin and DNP/anti-DNP immunoglobulin.

**22.** The method according to any of the preceding claims,
**characterised by** the targets being directly provided with a label.

**23.** The method according to any of the Claims 1 to 21,
**characterised by** a labelling of the targets being carried out through sandwich reactions or through sandwich hybridisations with the probes which interact with the targets and a labelled compound.

**24.** The method according to any of the Claims 1 to 21,
**characterised by** a labelling of the targets being carried out by adding a homopolymeric nucleotide sequence to the targets, under formation of a continuous sequence, followed by sandwich hybridisation with a labelled oligonucleotide which is complementary to the homopolymeric nucleotide sequence.

**25.** The method according to any of the preceding claims,
**characterised by** the interaction between the target and the probe being a hybridisation between two nucleotide sequences.

26. The method according to any of Claims 1 to 24,
**characterised by** the interaction between the target and the probe being a reaction between an antigen structure and the corresponding antibody or a hypervariable region thereof.

27. The method according to any of the preceding claims,
**characterised by** the interaction between the target and the probe being a reaction between a receptor and a corresponding ligand.

28. The method according to any of the preceding claims,
**characterised by** the detection of the presence of a precipitate on an array element being carried out by reflection, absorption or diffusion of a light beam, preferably a laser beam or a light-emitting diode, by the precipitate.

29. The method according to any of the Claims 1 to 27,
**characterised by** the detection of the presence of a precipitate on an array element being carried out electrically.

30. The method according to Claim 29,
**characterised by** the electrical detection being carried out by measurements of conductivity, capacity or potential.

31. The method according to any of the Claims 1 to 27,
**characterised by** the detection of the presence of a precipitate on an array element being carried out by autoradiography, fluorography and/or indirect autoradiography.

32. The method according to any of the Claims 1 to 27,
**characterised by** the presence of a precipitate on an array element being detected by scanning electron microscopy, electron probe microanalysis (EPMA), magneto-optic Kerr microscopy, magnetic force microscopy (MFM), atomic force microscopy (AFM), measurement of the mirage effect, scanning tunnelling microscopy (STM), and/or ultrasonic reflection tomography.

33. The method according to any of the preceding claims, comprising the following steps:

- detection of the time course of the formation of the precipitate on the array elements by taking pictures with a camera;
- conversion of the analog information contained in the pictures into a digital form;
- calculation of a virtual signal intensity for each array element on the basis of a curve function which describes the formation of the precipitate as a function of time;
- conversion of the virtual signal intensities into an artificial image which depicts the virtual signal intensities of all array elements.

34. A device to perform the method according to any of the claims 1 to 33, comprising:

a) an array substrate with a probe array,
b) a reaction chamber,
c) a device for the detection of a precipitate on an array element on which an interaction between targets and probes has occurred, and
d) a computer, which is programmed to:

- collect the signal intensities recorded by the detection device;
- guarantee the processing of the successively recorded signal intensities, so that the time course of the formation of the precipitate with time on an array element is determined and a virtual signal intensity is determined on the basis of a curve function which describes the formation of the precipitate as a function of time; and
- provide for, if required, the conversion of the virtual signal intensities into an analog picture.

35. The device according to Claim 34,
**characterised by** the detection device being a camera.

**36.** The device according to Claim 35,
**characterised by** the camera being a CCD or a CMOS camera.

**37.** The device according to any of the Claims 34 to 36,
**characterised by** the device also comprising a light source.

**38.** The device according to Claim 37,
**characterised by** the light source being selected from the group consisting of a laser, a light-emitting diode (LED), and a high pressure lamp.

**39.** The device according to any of the Claims 24 to 38,
**characterised by** the device being present as a highly integrated autonomous unit.

**Revendications**

**1.** Procédé de détection qualitative et/ou quantitative de cibles dans un échantillon par interactions moléculaires entre sondes et cibles sur des arrays de sondes, comportant les étapes suivantes :

a) réalisation d'un array de sondes comportant des sondes immobilisées à des endroits définis ;
b) interaction entre les cibles et les sondes disposées sur l'array de sondes ;
c) réalisation d'une réaction conduisant à un précipité aux éléments de l'array auxquels une interaction s'est produite ;
d) détection de l'évolution dans le temps de la formation du précipité sur les éléments de l'array sous la forme d'intensités de signaux ;
e) détermination d'une intensité virtuelle de signal pour un élément de l'array à l'aide d'une fonction de courbe qui décrit la formation de précipité en tant que fonction du temps.

**2.** Procédé selon la revendication 1,
**caractérisé en ce que** l'intensité virtuelle de signal pour un élément de l'array est déterminée en fonction de la pente d'une droite de régression qui décrit la formation de précipité en tant que fonction du temps.

**3.** Procédé selon la revendication 2,
**caractérisé en ce que** la droite de régression est déterminée dans la zone de croissance exponentielle de la formation de précipité dans le temps sur l'élément de l'array.

**4.** Procédé selon la revendication 2 ou 3,
**caractérisé en ce que** l'intensité virtuelle de signal pour un élément de l'array est définie par multiplication de l'intensité de signal détectée à un instant déterminé, de préférence l'intensité de signal de la dernière mesure, par la pente de la droite de régression déterminée pour l'élément de l'array, ainsi que par la durée de mesure à l'instant déterminé.

**5.** Procédé selon l'une quelconque des revendications précédentes,
**caractérisé en ce que** dans l'échantillon se présente une cible de référence de concentration connue qui interagit avec au moins une sonde de l'array de sondes.

**6.** Procédé selon l'une quelconque des revendications précédentes,
**caractérisé en ce que** pour la détection de l'évolution dans le temps de la formation de précipité aux éléments de l'array, les intensités de signal sont enregistrées au moins chaque minute, de préférence toutes les 30 secondes, de manière particulièrement préférée, toutes les 10 secondes.

**7.** Procédé selon l'une quelconque des revendications précédentes,
**caractérisé en ce que** la réaction conduisant à la formation d'un précipité aux éléments de l'array est la transformation d'un substrat soluble en un produit insoluble en présence d'un catalyseur couplé aux cibles.

**8.** Procédé selon la revendication 7,
**caractérisé en ce que** le catalyseur est une enzyme.

**9.** Procédé selon la revendication 7 ou 8,
**caractérisé en ce que** l'enzyme est choisie dans le groupe se composant de la peroxidase de raifort, la phosphatase alcaline et la glucose oxidase.

**10.** Procédé selon l'une quelconque des revendications 7 à 9,
**caractérisé en ce que** le substrat soluble est choisi dans le groupe se composant de 3,3'-diaminobenzidine, 4-chlore-1-naphthole, 3-amino-9-éthyle carbazole, p-diamine phénylène-HCl/pyrocatéchole, 3,3',5,5'-tétraméthylbenzidine, naphthole / pyronine, bromchlorindoylphosphate, bleu de nitrotétraazolium et méthosulfate de phénazine.

**11.** Procédé selon l'une quelconque des revendications 1 à 6,
**caractérisé en ce que** la réaction conduisant à la formation d'un précipité aux éléments de l'array est la transformation d'un substrat soluble en un précipité métallique.

**12.** Procédé selon la revendication 11,
**caractérisé en ce que** la réaction conduisant à la formation d'un précipité aux éléments de l'array est la réduction chimique d'un composé d'argent, de préférence de nitrate d'argent, de lactate d'argent, d'acétate d'argent ou de tartrate d'argent, en argent élémentaire.

**13.** Procédé selon la revendication 12,
**caractérisé en ce que** le réducteur est choisi dans le groupe se composant de formaldéhyde et d'hydroquinone.

**14.** Procédé selon l'une quelconque des revendications 11 à 13,
**caractérisé en ce que** la transformation d'un substrat soluble en un précipité métallique s'effectue en présence de clusters métalliques ou de particules métalliques colloïdales couplés aux cibles.

**15.** Procédé selon la revendication 14,
**caractérisé en ce que** la transformation d'un substrat soluble en un précipité métallique s'effectue en présence de clusters d'or ou de particules d'or colloïdales.

**16.** Procédé selon l'une quelconque des revendications 11 à 13,
**caractérisé en ce que** la transformation d'un substrat soluble en un précipité métallique s'effectue en présence de polyanions couplés aux cibles.

**17.** Procédé selon l'une quelconque des revendications 7 à 16,
**caractérisé en ce que** les catalyseurs ou clusters métalliques ou particules métalliques colloïdales ou polyanions sont couplés aux cibles avant, pendant ou après l'interaction avec les sondes.

**18.** Procédé selon l'une quelconque des revendications 7 à 17,
**caractérisé en ce que** le couplage des enzymes ou clusters métalliques ou particules métalliques colloïdales ou polyanions aux cibles s'effectue directement ou par des molécules d'ancrage couplées aux cibles.

**19.** Procédé selon la revendication 18,
**caractérisé en ce que** la molécule d'ancrage est choisie dans le groupe consistant en la streptavidine ou un anticorps.

**20.** Procédé selon l'une quelconque des revendications 1 à 6,
**caractérisé en ce que** la réaction conduisant à la formation d'un précipité aux éléments de l'array représente la liaison d'un partenaire de liaison spécifique avec une molécule d'ancrage couplée à la cible.

**21.** Procédé selon la revendication 20,
**caractérisé en ce que** les paires partenaire de liaison / molécule d'ancrage sont choisies dans le groupe se composant de biotine / avidine ou streptavidine ou anticorps anti-biotine, digoxigénine / immunoglobuline anti-digoxigénine, FITC / immunoglobuline anti-FITC et DNP / immunoglobuline anti-DNP.

**22.** Procédé selon l'une quelconque des revendications précédentes,
**caractérisé en ce que** les cibles sont pourvues directement d'un marquage.

**23.** Procédé selon l'une quelconque des revendications 1 à 21,
**caractérisé en ce qu'**un marquage des cibles s'effectue par des réactions sandwich ou des hybridations sandwich des sondes interagissant avec les cibles et un composé marqué.

**24.** Procédé selon l'une quelconque des revendications 1 à 21,
**caractérisé en ce qu'**un marquage des cibles s'effectue par ajout aux cibles d'une séquence nucléotidique homopolymère en formant une séquence continue, puis par hybridation sandwich avec un oligonucléotide marqué complémentaire à la séquence nucléotidique homopolymère.

**25.** Procédé selon l'une quelconque des revendications précédentes,
**caractérisé en ce que** l'interaction entre la cible et la sonde est une hybridation entre deux séquences nucléotidiques.

**26.** Procédé selon l'une quelconque des revendications 1 à 24,
**caractérisé en ce que** l'interaction entre la cible et la sonde est une réaction entre une structure antigénique et l'anticorps correspondant ou une partie hypervariable de celui-ci.

**27.** Procédé selon l'une quelconque des revendications précédentes,
**caractérisé en ce que** l'interaction entre la cible et la sonde est une réaction entre un récepteur et un ligand correspondant.

**28.** Procédé selon l'une quelconque des revendications précédentes,
**caractérisé en ce que** la détection de la présence d'un précipité sur un élément de l'array s'effectue par réflexion, absorption ou diffusion d'un rayon lumineux, de préférence d'un rayon laser ou d'une diode lumineuse, par le précipité.

**29.** Procédé selon l'une quelconque des revendications 1 à 27,
**caractérisé en ce que** la détection de la présence d'un précipité sur un élément de l'array s'effectue électriquement.

**30.** Procédé selon la revendication 29,
**caractérisé en ce que** la détection électrique s'effectue par des mesures de conductibilité, des mesures de capacité ou des mesures de potentiels.

**31.** Procédé selon l'une quelconque des revendications 1 à 27,
**caractérisé en ce que** la détection de la présence d'un précipité sur un élément de l'array s'effectue par autoradiographie, fluorographie et/ou autoradiographie indirecte.

**32.** Procédé selon l'une quelconque des revendications 1 à 27,
**caractérisé en ce que** la détection de la présence d'un précipité sur un élément de l'array s'effectue par microscopie électronique à balayage, microanalyse par sonde électronique (EPMA), microscopie Kerr magnéto-optique, microscopie à force magnétique (MFM), microscopie à force atomique (AFM), mesure de l'effet mirage, microscopie à balayage à effet tunnel (STM) et/ou tomographie de réflexion d'ultrasons.

**33.** Procédé selon l'une quelconque des revendications précédentes, comprenant les étapes suivantes :

- détection de l'évolution dans le temps de la formation de précipité aux éléments de l'array par la prise d'images par une caméra ;
- transformation en forme numérique de l'information analogique contenue dans les images ;
- calcul d'une intensité virtuelle de signal pour chaque élément de l'array à l'aide d'une fonction de courbe décrivant la formation de précipité en tant que fonction du temps ;
- transformation des intensités virtuelles de signal en une image synthétique qui représente les intensités virtuelles de signal de tous les éléments de l'array.

**34.** Dispositif pour appliquer le procédé selon l'une quelconque des revendications 1 à 33, comprenant :

a) un substrat d'array avec un array de sondes,
b) une chambre de réaction,

c) un dispositif pour la détection d'un précipité sur un élément de l'array auquel une interaction entre cibles et sondes s'est produite, et

d) un ordinateur programmé pour :

- collecter les intensités de signal enregistrées par le dispositif de détection ;
- assurer le traitement des intensités de signal enregistrées successivement de manière à ce que l'évolution dans le temps de la formation de précipité sur un élément de l'array est déterminée dans le temps et qu'une intensité virtuelle de signal est déterminée à l'aide d'une fonction de courbe qui décrit la formation de précipité en tant que fonction du temps ; et
- assurer, le cas échéant, la transformation des intensités virtuelles de signal en une image analogique.

35. Dispositif selon la revendication 34,
**caractérisé en ce que** le dispositif de détection est une caméra.

36. Dispositif selon la revendication 35,
**caractérisé en ce que** la caméra est une caméra CCD ou CMOS.

37. Dispositif selon l'une quelconque des revendications 34 à 36,
**caractérisé en ce que** le dispositif comprend en outre une source lumineuse.

38. Dispositif selon la revendication 37,
**caractérisé en ce que** la source lumineuse est choisie dans le groupe se composant d'un laser, d'une diode électroluminescente (DEL) et d'une lampe haute pression.

39. Dispositif selon l'une quelconque des revendications 24 à 38,
**caractérisé en ce que** le dispositif se présente en tant qu'unité autonome fortement intégrée.

Bildfolge für die Bestimmung
des zeitlichen Verlaufs

Kamera

Deckel

Dichtung

Verstärkungslösung

Arraysubstrat mit
Sondenarray

rote LED

Abb.1

Abb.2

**Abbildung 3**

**Abbildung 4**

| M  | 10 | 11 | 12 | 13 | 14 | 15 | 16 |    | M  |
|----|----|----|----|----|----|----|----|----|----|
| M  | 1  | 2  | 3  | 4  | 5  | 6  | 7  | 8  | 9  |
| 15 | 15 | 15 | 15 | 15 | 16 | 16 | 16 | 16 | 16 |
| 13 | 13 | 13 | 13 | 13 | 14 | 14 | 14 | 14 | 14 |
| 11 | 11 | 11 | 11 | 11 | 12 | 12 | 12 | 12 | 12 |
| 9  | 9  | 9  | 9  | 9  | 10 | 10 | 10 | 10 | 10 |
| 7  | 7  | 7  | 7  | 7  | 8  | 8  | 8  | 8  | 8  |
| 5  | 5  | 5  | 5  | 5  | 6  | 6  | 6  | 6  | 6  |
| 3  | 3  | 3  | 3  | 3  | 4  | 4  | 4  | 4  | 4  |
| M  | 1  | 1  | 1  | 1  | 2  | 2  | 2  | 2  | M  |

Abb.5

**Abbildung 6**

| M  | 10 | 10 | 10 | 10 | 10 | 10 | 10 | 10 | M  |
|----|----|----|----|----|----|----|----|----|----|
| M  | 9  | 9  | 9  | 9  | 9  | 9  | 9  | 9  | 9  |
| 8  | 8  | 8  | 8  | 8  | 8  | 8  | 8  | 8  | 8  |
| 7  | 7  | 7  | 7  | 7  | 7  | 7  | 7  | 7  | 7  |
| 6  | 6  | 6  | 6  | 6  | 6  | 6  | 6  | 6  | 6  |
| 5  | 5  | 5  | 5  | 5  | 5  | 5  | 5  | 5  | 5  |
| 4  | 4  | 4  | 4  | 4  | 4  | 4  | 4  | 4  | 4  |
| 3  | 3  | 3  | 3  | 3  | 3  | 3  | 3  | 3  | 3  |
| 2  | 2  | 2  | 2  | 2  | 2  | 2  | 2  | 2  | 2  |
| M  | 1  | 1  | 1  | 1  | 1  | 1  | 1  | 1  | M  |

Abb. 7

**Abbildung 8**

**Abbildung 9**

**Abbildung 10**

```
M   10  11  12  13  14  15  16          M
M    1   2   3   4   5   6   7   8   9
15  15  15  15  15  16  16  16  16  16
13  13  13  13  13  14  14  14  14  14
11  11  11  11  11  12  12  12  12  12
 9   9   9   9   9  10  10  10  10  10
 7   7   7   7   7   8   8   8   8   8
 5   5   5   5   5   6   6   6   6   6
 3   3   3   3   3   4   4   4   4   4
M    1   1   1   1   2   2   2   2   M
```

Abb.11

**Abbildung 12**

```
M    10   10   10   10   10   10   10   10   M
M     9    9    9    9    9    9    9    9    9
8     8    8    8    8    8    8    8    8    8
7     7    7    7    7    7    7    7    7    7
6     6    6    6    6    6    6    6    6    6
5     5    5    5    5    5    5    5    5    5
4     4    4    4    4    4    4    4    4    4
3     3    3    3    3    3    3    3    3    3
2     2    2    2    2    2    2    2    2    2
M     1    1    1    1    1    1    1    1    M
```

Abb.13

**Abbildung 14**

Abb. 15

| M | 10 | 10 | 10 | 10 | 10 | 10 | 10 | 10 | M |
|---|----|----|----|----|----|----|----|----|---|
| M | 9 | 9 | 9 | 9 | 9 | 9 | 9 | 9 | 9 |
| 8 | 8 | 8 | 8 | 8 | 8 | 8 | 8 | 8 | 8 |
| 7 | 7 | 7 | 7 | 7 | 7 | 7 | 7 | 7 | 7 |
| 6 | 6 | 6 | 6 | 6 | 6 | 6 | 6 | 6 | 6 |
| 5 | 5 | 5 | 5 | 5 | 5 | 5 | 5 | 5 | 5 |
| 4 | 4 | 4 | 4 | 4 | 4 | 4 | 4 | 4 | 4 |
| 3 | 3 | 3 | 3 | 3 | 3 | 3 | 3 | 3 | 3 |
| 2 | 2 | 2 | 2 | 2 | 2 | 2 | 2 | 2 | 2 |
| M | 1 | 1 | 1 | 1 | 1 | 1 | 1 | 1 | M |

Abb. 16

**Abbildung 17**

Abb. 18

Abb. 19a

Abb. 19b

Abb. 20